# EUROPEAN PATENT APPLICATION

(11) **EP 2 826 866 A1**
(43) Date of publication of application: **21.01.2015**
(21) Application number: 13306020.2
(22) Date of filing: 16.07.2013
(51) Int. Cl.: C12Q 1/68

(54) **MEST and MIR335/335* as biomarkers of mitochondrial diseases**

(71) Applicant: SANOFI, 75008 Paris (FR); Institut National de la Santé et de la Recherche Médicale, 75013 Paris (FR)
(72) Inventor: The designation of the inventor has not yet been filed
(74) Representative: Blot, Philippe Robert Emile

(57) **Abstract**

The present invention concerns an *in vitro* method of diagnosis or prognosis of a mitochondrial disease by detecting at least one of MEST, microRNA-335 or microRNA-335*, a precursor of said microRNA-335 and microRNA-335* and their use as biomarker of mitochondrial disease. The invention further concerns a ligand specifically binding to at least one of said biomarkers, for use for diagnosing or prognosing of a mitochondrial disease, a pair of primers suitable for amplifying a nucleic acid encoding for at least one of said biomarkers and the kit comprising such ligand and/or pair of primers.

SEQUENCE LISTING

<110>  SANOFI
       INSTITUT NATIONAL DE LA SANTE ET DE LA RECHERCHE MEDICALE
       (INSERM)

<120>  MEST AND MIR335/335* AS BIOMARKERS OF MITOCHONDRIAL DISEASES

<130>  BET13P0359

<160>  20

<170>  PatentIn version 3.5

<210>  1
<211>  2513
<212>  DNA
<213>  Homo sapiens

<400>  1
aaaagtcggt gcccactcgc tccgcgctgc cgcggcaacc agcacacccc ggcacctcct      60

ctgcggcagc tgcgcctcgc aagcgcagtg ccgcagcgca cgccggagtg gctgtagctg     120

cccggcgcgg cgccgccctg cgcgggctgt gggctgcggg ctgcgccccc gctgctggcc     180

agctctgcac ggctgcgggc tctgcggcgc ccggtgctct gcaacgctgc ggcgggcggc     240

atgggataac gcggccatgg tgcgccgaga tcgcctccgc aggatgaggg agtggtgggt     300

ccaggtgggg ctgctggccg tgcccctgct tgctgcgtac ctgcacatcc caccccctca     360

gctctcccct gcccttcact catggaagtc ttcaggcaag tttttcactt acaagggact      420

gcgtatcttc taccaagact ctgtgggtgt ggttggaagt ccagagatag ttgtgctttt     480

acacggtttt ccaacatcca gctacgactg gtacaagatt tgggaaggtc tgaccttgag     540

gtttcatcgg gtgattgccc ttgatttctt aggctttggc ttcagtgaca aaccgagacc     600

acatcactat tccatatttg agcaggccag catcgtggaa gcgcttttgc ggcatctggg     660

gctccagaac cgcaggatca accttctttc tcatgactat ggagatattg ttgctcagga     720

gcttctctac aggtacaagc agaatcgatc tggtcggctt accataaaga gtctctgtct     780

gtcaaatgga ggtatctttc ctgagactca ccgtccactc cttctccaaa agctactcaa     840

agatggaggt gtgctgtcac ccatcctcac acgactgatg aacttctttg tattctctcg     900

aggtctcacc ccagtctttg ggccgtatac tcggccctct gagagtgagc tgtgggacat     960

gtgggcaggg atccgcaaca atgacgggaa cttagtcatt gacagtctct tacagtacat    1020

caatcagagg aagaagttca gaaggcgctg ggtgggagct cttgcctctg taactatccc    1080

cattcatttt atctatgggc cattggatcc tgtaaatccc tatccagagt ttttggagct     1140

gtacaggaaa acgctgccgc ggtccacagt gtcgattctg gatgaccaca ttagccacta    1200

tccacagcta gaggatccca tgggcttctt gaatgcatat atgggcttca tcaactcctt    1260

ctgagctgga aagagtagct tccctgtatt acctccccta ctcccttatg tgttgtgtat    1320

tccacttagg aagaaatgcc caaaagaggt cctggccatc aaacataatt ctctcacaaa 1380

gtccacttta ctcaaattgg tgaacagtgt ataggaagaa gccagcagga gctctgacta 1440

aggttgacat aatagtccac ctcccattac tttgatatct gatcaaatgt atagacttgg 1500

ctttgttttt tgtgctatta ggaaattctg atgagcatta ctattcactg atgcagaaag 1560

acgttctttt gcataaaaga ctttttttta acactttgga cttctctgaa atatttagaa 1620

gtgctaattt ctggcccacc cccaacagga attctatagt aaggaggagg agaagggggg 1680

ctccttccct ctcctcgaat gacgttatgg gcacatgcct tttaaaagtt ctttaagcaa 1740

cacagagctg agtcctcttt gtcatacctt tggatttagt gtttcatcag ctgtttttag 1800

ttataaacat tttgttaaaa tagatattgg tttaaatgat acagtatttt aggtatgatt 1860

taagactatg atttacctat acattatata tattttataa agatactaaa ccagcatacc 1920

cttactctgc cagagtagtg aagctaatta aacacatttg gtttctgaat aaattgaact 1980

aaatccaaac tatttcctaa aatcacagga cattaaggac caatagcatc tgtgccagag 2040

atgtactgtt attagctggg aagaccaatt ctaacagcaa ataacagtct gagactcctc 2100

atacctcagt ggttagaagc atgtctctct tgagctacag tagaggggaa gggattgttg 2160

tgtagtcaag tcaccatgct gaatgtacac tgattccttt atgatgactg cttaactccc 2220

cactgcctgt cccagagagg ctttccaatg tagctcagta attcctgtta ctttacagac 2280

aggaaagttc cagaaacttt aagaacaaac tctgaaagac ctatgagcaa atggtgctga 2340

atactttttt tttaaagcca catttcattg tcttagtcaa agcaggatta ttaagtgatt 2400

atttaaaatt cgttttttta aattagcaac ttcaagtata acaactttga aactggaata 2460

agtgtttatt ttctattaat aaaaatgaat tgtgacaaaa gtggaaaaaa aaa 2513

<210> 2
<211> 2452
<212> DNA
<213> Homo sapiens

<400> 2
   ggcgctgacg cctggcaggg agaaggcggc agcacatgct gggctcgggg gcgatgggct 60
   tgtgcgcgga cctggcgacg ctctagcccc gagccgcgta ttcgtggccg ggtcctccct 120
   gggaacaggg tgaaggccga gaacctctgg cctcaggaag cgcatgcgca accggttctc 180
   cgaaacatgg agtcctgtag gcaaggtctt acctgaatca ggatgaggga gtggtgggtc 240
   caggtggggc tgctggccgt gcccctgctt gctgcgtacc tgcacatccc accccctcag 300
   ctctcccctg cccttcactc atggaagtct tcaggcaagt ttttcactta caagggactg 360
   cgtatcttct accaagactc tgtgggtgtg gttggaagtc cagagatagt tgtgctttta 420
   cacggttttc caacatccag ctacgactgg tacaagattt gggaaggtct gaccttgagg 480
   tttcatcggg tgattgccct tgatttctta ggctttggct tcagtgacaa accgagacca 540
   catcactatt ccatatttga gcaggccagc atcgtggaag cgcttttgcg gcatctgggg 600
   ctccagaacc gcaggatcaa ccttctttct catgactatg gagatattgt tgctcaggag 660
   cttctctaca ggtacaagca gaatcgatct ggtcggctta ccataaagag tctctgtctg 720
   tcaaatggag gtatctttcc tgagactcac cgtccactcc ttctccaaaa gctactcaaa 780
   gatggaggtg tgctgtcacc catcctcaca cgactgatga acttctttgt attctctcga 840
   ggtctcaccc cagtctttgg gccgtatact cggccctctg agagtgagct gtgggacatg 900
   tgggcaggga tccgcaacaa tgacgggaac ttagtcattg acagtctctt acagtacatc 960
   aatcagagga agaagttcag aaggcgctgg gtgggagctc ttgcctctgt aactatcccc 1020
   attcatttta tctatgggcc attggatcct gtaaatccct atccagagtt tttggagctg 1080
   tacaggaaaa cgctgccgcg gtccacagtg tcgattctgg atgaccacat tagccactat 1140
   ccacagctag aggatcccat gggcttcttg aatgcatata tgggcttcat caactccttc 1200
   tgagctggaa agagtagctt ccctgtatta cctcccctac tcccttatgt gttgtgtatt 1260
   ccacttagga agaaatgccc aaaagaggtc ctggccatca aacataattc tctcacaaag 1320
   tccactttac tcaaattggt gaacagtgta taggaagaag ccagcaggag ctctgactaa 1380
   ggttgacata atagtccacc tcccattact ttgatatctg atcaaatgta tagacttggc 1440
   tttgtttttt gtgctattag gaaattctga tgagcattac tattcactga tgcagaaaga 1500
   cgttcttttg cataaaagac ttttttttaa cactttggac ttctctgaaa tatttagaag 1560
   tgctaatttc tggcccaccc ccaacaggaa ttctatagta aggaggagga gaaggggggc 1620
   tccttccctc tcctcgaatg acgttatggg cacatgcctt ttaaaagttc tttaagcaac 1680
   acagagctga gtcctctttg tcataccttt ggatttagtg tttcatcagc tgtttttagt 1740
   tataaacatt ttgttaaaat agatattggt ttaaatgata cagtatttta ggtatgattt 1800
   aagactatga tttacctata cattatatat attttataaa gatactaaac cagcataccc 1860
   ttactctgcc agagtagtga agctaattaa acacatttgg tttctgaata aattgaacta 1920
   aatccaaact atttcctaaa atcacaggac attaaggacc aatagcatct gtgccagaga 1980
   tgtactgtta ttagctggga agaccaattc taacagcaaa taacagtctg agactcctca 2040
   tacctcagtg gttagaagca tgtctctctt gagctacagt agaggggaag ggattgttgt 2100
   gtagtcaagt caccatgctg aatgtacact gattccttta tgatgactgc ttaactcccc 2160
   actgcctgtc ccagagaggc tttccaatgt agctcagtaa ttcctgttac tttacagaca 2220
   ggaaagttcc agaaacttta agaacaaact ctgaaagacc tatgagcaaa tggtgctgaa 2280
   tacttttttt ttaaagccac atttcattgt cttagtcaaa gcaggattat taagtgatta 2340
   tttaaaattc gtttttttaa attagcaact tcaagtataa caactttgaa actggaataa 2400
   gtgtttattt tctattaata aaaatgaatt gtgacaaaag tggaaaaaaa aa 2452

<210> 3
<211> 2389
<212> DNA
<213> Homo sapiens

<400> 3
gggcgctgct gtggccaggc gtctggcatg ctgaccacgt cgcgctgctg taaaggaaac 60

ctgccccgcg cagcggcggt ggctggagcg ggagaaaccg gactttgtgc aactttggcc 120

atagtggcca tcccatgaat ctgtttacta gcttggtgga tgagggagtg gtgggtccag 180

gtggggctgc tggccgtgcc cctgcttgct gcgtacctgc acatcccacc ccctcagctc 240

tcccctgccc ttcactcatg gaagtcttca ggcaagtttt tcacttacaa gggactgcgt 300

atcttctacc aagactctgt gggtgtggtt ggaagtccag agatagttgt gcttttacac 360

ggttttccaa catccagcta cgactggtac aagatttggg aaggtctgac cttgaggttt 420

catcgggtga ttgcccttga tttcttaggc tttggcttca gtgacaaacc gagaccacat 480

cactattcca tatttgagca ggccagcatc gtggaagcgc ttttgcggca tctggggctc 540

cagaaccgca ggatcaacct tctttctcat gactatggag atattgttgc tcaggagctt 600

ctctacaggt acaagcagaa tcgatctggt cggcttacca taaagagtct ctgtctgtca 660

aatggaggta tctttcctga gactcaccgt ccactccttc tccaaaagct actcaaagat 720

ggaggtgtgc tgtcacccat cctcacacga ctgatgaact tctttgtatt ctctcgaggt 780

ctcaccccag tctttgggcc gtatactcgg ccctctgaga gtgagctgtg ggacatgtgg 840

gcagggatcc gcaacaatga cgggaactta gtcattgaca gtctcttaca gtacatcaat 900

cagaggaaga agttcagaag gcgctgggtg ggagctcttg cctctgtaac tatccccatt 960

cattttatct atgggccatt ggatcctgta aatccctatc cagagttttt ggagctgtac 1020

aggaaaacgc tgccgcggtc cacagtgtcg attctggatg accacattag ccactatcca 1080

cagctagagg atcccatggg cttcttgaat gcatatatgg gcttcatcaa ctccttctga 1140

gctggaaaga gtagcttccc tgtattacct cccctactcc cttatgtgtt gtgtattcca 1200

cttaggaaga aatgcccaaa agaggtcctg gccatcaaac ataattctct cacaaagtcc 1260

actttactca aattggtgaa cagtgtatag gaagaagcca gcaggagctc tgactaaggt 1320

tgacataata gtccacctcc cattactttg atatctgatc aaatgtatag acttggcttt 1380

gttttttgtg ctattaggaa attctgatga gcattactat tcactgatgc agaaagacgt 1440

tcttttgcat aaaagacttt tttttaacac tttggacttc tctgaaatat ttagaagtgc 1500

taatttctgg cccaccccca acaggaattc tatagtaagg aggaggagaa ggggggctcc 1560

ttccctctcc tcgaatgacg ttatgggcac atgcctttta aaagttcttt aagcaacaca 1620

gagctgagtc ctctttgtca tacctttgga tttagtgttt catcagctgt ttttagttat 1680

aaacattttg ttaaaataga tattggttta aatgatacag tattttaggt atgatttaag 1740

actatgattt acctatacat tatatatatt ttataaagat actaaaccag cataccctta 1800

ctctgccaga gtagtgaagc taattaaaca catttggttt ctgaataaat tgaactaaat 1860

ccaaactatt tcctaaaatc acaggacatt aaggaccaat agcatctgtg ccagagatgt 1920

actgttatta gctgggaaga ccaattctaa cagcaaataa cagtctgaga ctcctcatac 1980

ctcagtggtt agaagcatgt ctctcttgag ctacagtaga ggggaaggga ttgttgtgta 2040

gtcaagtcac catgctgaat gtacactgat tcctttatga tgactgctta actccccact 2100

gcctgtccca gagaggcttt ccaatgtagc tcagtaattc ctgttacttt acagacagga 2160

aagttccaga aactttaaga acaaactctg aaagacctat gagcaaatgg tgctgaatac 2220

ttttttttta aagccacatt tcattgtctt agtcaaagca ggattattaa gtgattattt 2280

aaaattcgtt tttttaaatt agcaacttca agtataacaa ctttgaaact ggaataagtg 2340

tttattttct attaataaaa atgaattgtg acaaaagtgg aaaaaaaaa 2389

<210> 4
<211> 335
<212> PRT
<213> Homo sapiens

<400> 4

<210>  5
<211>  23
<212>  RNA
<213>  Homo sapiens

<400>  5

ucaagagcaa uaacgaaaaa ugu                                          23


<210>  6
<211>  22
<212>  RNA
<213>  Homo sapiens

<400>  6
uuuuucauua uugcuccuga cc                                           22


<210>  7
<211>  94
<212>  RNA
<213>  Homo sapiens

<400>  7
uguuuugagc gggggucaag agcaauaacg aaaaauguuu gucauaaacc guuuuucauu    60

auugcuccug accuccucuc auuugcuaua uuca                              94


<210>  8
<211>  983
<212>  DNA
<213>  Homo sapiens

<400>  8
ggatgaggga gtggtgggtc caggtggggc tgctggccgt gcccctgctt gctgcgtacc    60

tgcacatccc accccctcag ctctcccctg cccttcactc atggaagtct tcaggcaagt   120

tttcacttta caagggactg cgtatcttct accaagactc tgtgggtgtg gttggaagtc   180

cagagatagt tgtgctttta cacggttttc caacatccag ctacgactgg tacaagattt   240

gggaaggtct gaccttgagg tttcatcggg tgattgccct tgatttctta ggctttggct   300

tcagtgacaa accgagacca catcactatt ccatatttga gcaggccagc atcgtggaag   360

cgcttttgcg gcatctgggg ctccagaacc gcaggatcaa ccttctttct catgactatg   420

gagatattgt tgctcaggag cttctctaca ggtacaagca gaatcgatct ggtcggctta   480

ccataaagag tctctgtctg tcaaatggag gtatctttcc tgagactcac cgtccactcc   540

ttctccaaaa gctactcaaa gatggaggtg tgctgtcacc catcctcaca cgactgatga   600

acttctttgt attctctcga ggtctcaccc cagtctttgg gccgtatact cggccctctg   660

agagtgagct gtgggacatg tgggcaggga tccgcaacaa tgacgggaac ttagtcattg   720

acagtctctt acagtacatc aatcagagga agaagttcag aaggcgctgg gtgggagctc   780

ttgcctctgt aactatcccc attcatttta tctatgggcc attggatcct gtaaatccct   840

atccagagtt tttggagctg tacaggaaaa cgctgccgcg gtccacagtg tcgattctgg   900

atgaccacat tagccactat ccacagctag aggatcccat gggcttcttg aatgcatata   960

tgggcttcat caactccttc tga                                          983

<210>  9
<211>  37
<212>  PRT
<213>  Homo sapiens

<400>  9

Met Arg Glu Trp Trp Val Gln Val Gly Leu Leu Ala Val Pro Leu Leu
1               5                   10                  15

Ala Ala Tyr Leu His Ile Pro Pro Pro Gln Leu Ser Pro Ala Leu His
            20                  25                  30

Ser Trp Lys Ser Ser
        35

<210>  10
<211>  157
<212>  PRT
<213>  Homo sapiens

<400>  10

Asp Ser Val Gly Val Val Gly Ser Pro Glu Ile Val Val Leu Leu His
1               5                   10                  15

Gly Phe Pro Thr Ser Ser Tyr Asp Trp Tyr Lys Ile Trp Glu Gly Leu
            20                  25                  30

Thr Leu Arg Phe His Arg Val Ile Ala Leu Asp Phe Leu Gly Phe Gly
        35                  40                  45

Phe Ser Asp Lys Pro Arg Pro His His Tyr Ser Ile Phe Glu Gln Ala
    50                  55                  60

Ser Ile Val Glu Ala Leu Leu Arg His Leu Gly Leu Gln Asn Arg Arg
65                  70                  75                  80

Ile Asn Leu Leu Ser His Asp Tyr Gly Asp Ile Val Ala Gln Glu Leu
                85                  90                  95

Leu Tyr Arg Tyr Lys Gln Asn Arg Ser Gly Arg Leu Thr Ile Lys Ser
            100                 105                 110

Leu Cys Leu Ser Asn Gly Gly Ile Phe Pro Glu Thr His Arg Pro Leu
        115                 120                 125

Leu Leu Gln Lys Leu Leu Lys Asp Gly Gly Val Leu Ser Pro Ile Leu
    130                 135                 140

Thr Arg Leu Met Asn Phe Phe Val Phe Ser Arg Gly Leu
145                 150                 155

<210>  11
<211>  2471
<212>  DNA
<213>  Homo sapiens

<400>  11
aaaagtcggt gcccactcgc tccgcgctgc cgcggcaacc agcacacccc ggcacctcct      60

ctgcggcagc tgcgcctcgc aagcgcagtg ccgcagcgca cgccggagtg gctgtagctg     120

cccggcgcgg cgccgccctg cgcgggctgt gggctgcggg ctgcgccccc gctgctggcc     180

agctctgcac ggctgcgggc tctgcggcgc ccggtgctct gcaacgctgc ggcgggcggc     240

atgggataac gcggccatgg tgcgccgaga tcgcctccgc aggatgaggg agtggtgggt     300

ccaggtgggg ctgctggccg tgcccctgct tgctgcgtac ctgcacatcc caccccctca     360

gctctcccct gcccttcact catggaagtc ttcagactct gtgggtgtgg ttggaagtcc     420

agagatagtt gtgcttttac acggttttcc aacatccagc tacgactggt acaagatttg     480

ggaaggtctg accttgaggt ttcatcgggt gattgccctt gatttcttag gctttggctt     540

cagtgacaaa ccgagaccac atcactattc catatttgag caggccagca tcgtggaagc     600

gcttttgcgg catctggggc tccagaaccg caggatcaac cttctttctc atgactatgg     660

agatattgtt gctcaggagc ttctctacag gtacaagcag aatcgatctg gtcggcttac     720

cataaagagt ctctgtctgt caaatggagg tatctttcct gagactcacc gtccactcct     780

tctccaaaag ctactcaaag atggaggtgt gctgtcaccc atcctcacac gactgatgaa     840

cttctttgta ttctctcgag gtctcacccc agtctttggg ccgtatactc ggccctctga     900

gagtgagctg tgggacatgt gggcagggat ccgcaacaat gacgggaact tagtcattga     960

cagtctctta cagtacatca atcagaggaa gaagttcaga aggcgctggg tgggagctct    1020

tgcctctgta actatcccca ttcattttat ctatgggcca ttggatcctg taaatcccta    1080

tccagagttt ttggagctgt acaggaaaac gctgccgcgg tccacagtgt cgattctgga    1140

tgaccacatt agccactatc cacagctaga ggatcccatg ggcttcttga atgcatatat    1200

gggcttcatc aactccttct gagctggaaa gagtagcttc cctgtattac ctcccctact    1260

cccttatgtg ttgtgtattc cacttaggaa gaaatgccca aaagaggtcc tggccatcaa    1320

acataattct ctcacaaagt ccactttact caaattggtg aacagtgtat aggaagaagc    1380

cagcaggagc tctgactaag gttgacataa tagtccacct cccattactt tgatatctga    1440

tcaaatgtat agacttggct ttgttttttg tgctattagg aaattctgat gagcattact    1500

attcactgat gcagaaagac gttcttttgc ataaaagact tttttttaac actttggact    1560

tctctgaaat atttagaagt gctaatttct ggcccacccc caacaggaat tctatagtaa    1620

ggaggaggag aaggggggct ccttccctct cctcgaatga cgttatgggc acatgccttt 1680

taaaagttct ttaagcaaca cagagctgag tcctctttgt catacctttg gatttagtgt 1740

ttcatcagct gtttttagtt ataaacattt tgttaaaata gatattggtt taaatgatac 1800

agtattttag gtatgattta agactatgat ttacctatac attatatata ttttataaag 1860

atactaaacc agcataccct tactctgcca gagtagtgaa gctaattaaa cacatttggt 1920

ttctgaataa attgaactaa atccaaacta tttcctaaaa tcacaggaca ttaaggacca 1980

atagcatctg tgccagagat gtactgttat tagctgggaa gaccaattct aacagcaaat 2040

aacagtctga gactcctcat acctcagtgg ttagaagcat gtctctcttg agctacagta 2100

gaggggaagg gattgttgtg tagtcaagtc accatgctga atgtacactg attcctttat 2160

gatgactgct taactcccca ctgcctgtcc cagagaggct ttccaatgta gctcagtaat 2220

tcctgttact ttacagacag gaaagttcca gaaactttaa gaacaaactc tgaaagacct 2280

atgagcaaat ggtgctgaat actttttttt taaagccaca tttcattgtc ttagtcaaag 2340

caggattatt aagtgattat ttaaaattcg tttttttaaa ttagcaactt caagtataac 2400

aactttgaaa ctggaataag tgtttatttt ctattaataa aaatgaattg tgacaaaagt 2460

ggaaaaaaaa a 2471

<210> 12
<211> 2350
<212> DNA
<213> Homo sapiens

<400> 12
ggcgctgacg cctggcaggg agaaggcggc agcacatgct gggctcgggg gcgatgggct 60

tgtgcgcgga cctggcgacg ctctagcccc gagccgcgta ttcgtggccg ggtcctccct 120

gggaacaggg tgaaggccga gaacctctgg cctcaggaag cgcatgcgca accggttctc 180

cgaaacatgg agtcctgtag gcaaggtctt acctgaatca ggatgaggga gtggtgggtc 240

caggtggggc tgctggccgt gcccctgctt gctgcgtacc tgcacatccc accccctcag 300

ctctcccctg cccttcactc atggaagtct tcaggcaagt ttttcactta caagggactg 360

cgtatcttct accaagactc tgtgggtgtg gttggaagtc cagagatagt tgtgctttta 420

cacggttttc caacatccag ctacgactgg tacaagattt gggaaggtct gaccttgagg 480

tttcatcggg tgattgccct tgatttctta ggctttggct tcagtgacaa accgagacca 540

catcactatt ccatatttga gcaggccagc atcgtggaag cgcttttgcg gcatctgggg 600

ctccagaacc gcaggatcaa ccttctttct catgactatg gagatattgt tgctcaggag 660

cttctctaca ggtacaagca gaatcgatct ggtcggctta ccataaagag tctctgtctg 720

tcaaatggag gtatctttcc tgagactcac cgtccactcc ttctccaaaa gctactcaaa 780

gatggaggtg tgctgtcacc catcctcaca cgactgatga acttctttgt attctctcga 840

ggtctcttac agtacatcaa tcagaggaag aagttcagaa ggcgctgggt gggagctctt 900

gcctctgtaa ctatccccat tcattttatc tatgggccat tggatcctgt aaatccctat 960

ccagagtttt tggagctgta caggaaaacg ctgccgcggt ccacagtgtc gattctggat 1020

gaccacatta gccactatcc acagctagag gatcccatgg gcttcttgaa tgcatatatg 1080

ggcttcatca actccttctg agctggaaag agtagcttcc ctgtattacc tcccctactc 1140

ccttatgtgt tgtgtattcc acttaggaag aaatgcccaa aagaggtcct ggccatcaaa 1200

cataattctc tcacaaagtc cactttactc aaattggtga acagtgtata ggaagaagcc 1260

agcaggagct ctgactaagg ttgacataat agtccacctc ccattacttt gatatctgat 1320

caaatgtata gacttggctt tgttttttgt gctattagga aattctgatg agcattacta 1380

ttcactgatg cagaaagacg ttcttttgca taaaagactt ttttttaaca ctttggactt 1440

ctctgaaata tttagaagtg ctaatttctg gcccaccccc aacaggaatt ctatagtaag 1500

gaggaggaga aggggggctc cttccctctc ctcgaatgac gttatgggca catgcctttt 1560

aaaagttctt taagcaacac agagctgagt cctctttgtc atacctttgg atttagtgtt 1620

tcatcagctg tttttagtta taaacatttt gttaaaatag atattggttt aaatgataca 1680

gtattttagg tatgatttaa gactatgatt tacctataca ttatatatat tttataaaga 1740

tactaaacca gcataccctt actctgccag agtagtgaag ctaattaaac acatttggtt 1800

tctgaataaa ttgaactaaa tccaaactat ttcctaaaat cacaggacat taaggaccaa 1860

tagcatctgt gccagagatg tactgttatt agctgggaag accaattcta acagcaaata 1920

acagtctgag actcctcata cctcagtggt tagaagcatg tctctcttga gctacagtag 1980

aggggaaggg attgttgtgt agtcaagtca ccatgctgaa tgtacactga ttcctttatg 2040

atgactgctt aactccccac tgcctgtccc agagaggctt tccaatgtag ctcagtaatt 2100

cctgttactt tacagacagg aaagttccag aaactttaag aacaaactct gaaagaccta 2160

tgagcaaatg gtgctgaata cttttttttt aaagccacat ttcattgtct tagtcaaagc 2220

aggattatta agtgattatt taaaattcgt ttttttaaat tagcaacttc aagtataaca 2280

actttgaaac tggaataagt gtttattttc tattaataaa aatgaattgt gacaaaagtg 2340

gaaaaaaaaa 2350

<210> 13
<211> 113
<212> DNA
<213> Homo sapiens

<400> 13
ggatgaggga gtggtgggtc caggtggggc tgctggccgt gcccctgctt gctgcgtacc 60

tgcacatccc accccctcag ctctcccctg cccttcactc atggaagtct tca 113

<210> 14
<211> 469
<212> DNA
<213> Homo sapiens

<400> 14
gactctgtgg gtgtggttgg aagtccagag atagttgtgc ttttacacgg ttttccaaca 60

tccagctacg actggtacaa gatttgggaa ggtctgacct tgaggtttca tcgggtgatt 120

gcccttgatt tcttaggctt tggcttcagt gacaaaccga gaccacatca ctattccata 180

tttgagcagg ccagcatcgt ggaagcgctt ttgcggcatc tggggctcca gaaccgcagg 240

atcaaccttc tttctcatga ctatggagat attgttgctc aggagcttct ctacaggtac 300

aagcagaatc gatctggtcg gcttaccata aagagtctct gtctgtcaaa tggaggtatc 360

tttcctgaga ctcaccgtcc actccttctc caaaagctac tcaaagatgg aggtgtgctg 420

tcacccatcc tcacacgact gatgaacttc tttgtattct ctcgaggtc 469

<210> 15
<211> 257
<212> DNA
<213> Homo sapiens

<400> 15
tcttacagta catcaatcag aggaagaagt tcagaaggcg ctgggtggga gctcttgcct 60

ctgtaactat ccccattcat tttatctatg ggccattgga tcctgtaaat ccctatccag 120

agtttttgga gctgtacagg aaaacgctgc cgcggtccac agtgtcgatt ctggatgacc 180

acattagcca ctatccacag ctagaggatc ccatgggctt cttgaatgca tatatgggct 240

tcatcaactc cttctga 257

<210> 16
<211> 25
<212> DNA
<213> Artificial sequence

<220>
<223> Primer probe

<400> 16
cccaccccca acaggaattc tatag 25

<210> 17
<211> 25
<212> DNA
<213> Artificial sequence

<220>
<223> Primer, probe

<400>  17
aaatgtttca ttgtgggagc agaca                                              25


<210>  18
<211>  25
<212>  DNA
<213>  Artificial sequence

<220>
<223>  Primer, probe

<400>  18
tcgcttgcgc aggatgagag agtgg                                              25


<210>  19
<211>  25
<212>  DNA
<213>  Artificial sequence

<220>
<223>  Primer, probe

<400>  19
gtgaacggat ttggccgtat tgggc                                              25


<210>  20
<211>  84
<212>  PRT
<213>  Homo sapiens

<400>  20

Leu Gln Tyr Ile Asn Gln Arg Lys Lys Phe Arg Arg Arg Trp Val Gly
1               5                   10                  15


Ala Leu Ala Ser Val Thr Ile Pro Ile His Phe Ile Tyr Gly Pro Leu
            20                  25                  30


Asp Pro Val Asn Pro Tyr Pro Glu Phe Leu Glu Leu Tyr Arg Lys Thr
        35                  40                  45


Leu Pro Arg Ser Thr Val Ser Ile Leu Asp Asp His Ile Ser His Tyr
    50                  55                  60


Pro Gln Leu Glu Asp Pro Met Gly Phe Leu Asn Ala Tyr Met Gly Phe
65                  70                  75                  80


Ile Asn Ser Phe

## Description

The present invention concerns an *in vitro* method of diagnosis or prognosis of a mitochondrial disease by detecting at least one of MEST, microRNA-335 or microRNA-335* and their use as biomarker of mitochondrial disease. The invention further concerns a ligand specifically binding to at least one of said biomarkers, for use for diagnosing or prognosing of a mitochondrial disease, a pair of primers suitable for amplifying a nucleic acid encoding for at least one of said biomarkers and the kit comprising such ligand and/or pair of primers.

### BACKGROUND TO THE INVENTION

Mitochondria are organelles present in cells and deeply involved in energy production. Disturbances in mitochondria are described as being responsible of a large panel of disease. These diseases are collectively called as "mitochondrial disease". The mitochondrial disease is classified in various ways by biochemical abnormalities, clinical symptoms or types of DNA abnormalities.

Among those mitochondrial diseases, Inherited Optic Neuropathies (IONs) are degenerative diseases of the retinal ganglion cells (RGCs). IONs are transmitted either maternally by mitochondrial DNA (Leber IONs, with acute evolution), or dominantly by nuclear genes, such as OPA1 which encodes intra-mitochondrial proteins (DOA - Dominant Optic Atrophy, with chronic evolution). Vision loss is due to optic nerve fiber atrophy consecutively to the RGCs apoptosis (Amati-Bonneau, P. et al. 2009. Int J Biochem Cell Biol 4tz 1855-1865*;* Lenaers, G. et al 2009. Int J Biochem Cell Biol 4L t866-L874*).* This condition is due to mitochondrial dysfunction mediating the death of optic nerve fibers. Dominant optic atrophy is mainly associated with mutation of the OPA1 gene and Leber ION with mutations in mitochondrial genome *(*Olichon, et al. 2007 J Cell Physiol 211: 423-430*;* Harding AE, et al. Am J Hum Genet 1995; 57: 77-86)*.*

IONs are at the intersection of several medical fields: (i) With ophthalmology: RGC loss causes blindness and IONs share some deficiencies with glaucoma, particularly the NTG sub-class (Non-Tension Glaucoma: 1/3 of all cases) for which a mitochondrial defect is proposed *(*Lee, D. A., and Higginbotham, E. J. (2005) Am. J. Health Syst. Pharm. 62, 691-699); (ii) With neurology: RGCs are neurons of the CNS, sharing many properties with other neurons. In Parkinson and Huntington diseases, mitochondrial dynamic dysfunction was described as contributing to the pathophysiology; *(*Mandemakers W, et al (2007) J Cell Sci 120: 1707-1716*)* (iii) With mitochondriopathies: the domain contains hundreds of rare diseases, and the most common disease, aging.

OPA1, which encodes dynamin related proteins involved in membrane dynamics, is responsible for the maintenance of the integrity of the mitochondrial inner membrane (MIM) and the structure of the cristae (Olichon, A. et al. 2003. J Biol Chem 278: 7743-7746*).* As a consequence mutation of OPA1 leads to the alteration of a wide variety of mitochondrial processes such as fusion of the mitochondrial network, segregation of cytochrome c, oxidative phosphorylation (OXPHOS), membrane potential maintenance, ROS production, calcium buffering and maintenance of the mitochondrial genome integrity *(*Chevrollier, A., et al. 2008. Ann Neurol 63: 794-798*;* Dayanithi, G., et al. 2010, Ophthalmic Genet 31: 53-65*;* Elachouri, G. et al. 2011. Genome Res 21: 12-20*;* Olichon, A., Cell Death Differ 2007 14: 682-692).

The mitochondrial dynamics is an overlapping interplay between fusion, fission, mitophagy and mobility *(*Twig, G., et al. 2008. Embo J. 27: 433-446*).* This dynamics is implicated, for example, in cell adaptation to energy requirements and in maintaining mitochondria integrity related to physiological and environment conditions. Emergence of deregulation of keys effectors of mitochondrial dynamics in several diseases, more particularly in neurodegenerative diseases such as Alzheimer Disease (AD), Parkinson Disease, Huntington Disease (Chen Hsiuchen and Chan David C. 2009. Human Molecular Genetics, Vol. 18, Review Issue 2, R169-R1768) Type 2 diabetes (Zorzanoa Antonio, et al. 2009. The International Journal of Biochemistry & Cell Biology 41: 1846-1854*)* and Friedreich ataxia for example (Rötig A, et al. 1997. Nat Genet.: 17:215-7)*,* indicates implication of mitochondrial dynamics in the etiology of these pathologies. As examples, (i) parkin is described to regulate mitochondrial dynamics by inhibiting mitochondrial fusion through the ubiquitination and degradation of mitofusins, others key mitochondrial proteins for fusion; (ii) exposure of cerebrocortical neurons in culture to Aβ results in the formation of amounts of S-Nitrosylation of Drp1 (a key fission protein) relatively similar to those found in human AD brains, leading mitochondrial fragmentation; (iii) Type 2 diabetic patients show a reduction in mitofusin-2 expression in skeletal muscle compared to control subjects and mitochondrial size is reduced in skeletal muscle of obese and type 2 diabetic patients compared to lean subjects.

Important features of mitochondrial disorders remain unexplained, more particularly, the marked incomplete penetrance of inherited mutations, even when patients shares similar or identical mutations. Such feature indicates that secondary factors modulate the risk to develop pathology as well as the grade of the pathology *(Yu-Wai-*Mana Patrick, et al. 2011. Prog Retin Eye Res; 30: 81-114*;* Yu-Wai-Man P, et al. 2009. J Med Genet.: 46:145-158)*.* For example, only 50% of male and 10% of female LHON carriers lost vision in their lifetime and only some patients with MFN2 will develop visual default. Similarly incomplete penetrance in DOA families implies that other factors are potentiating the deleterious effects of the OPA1 mutations. Identification of such factors is promising in terms of biomarkers and targets for pharmacological compounds.

The inventors have surprisingly shown that MEST (SEQ ID NO: 1, SEQ ID NO: 2 or SEQ ID NO: 3) and the MEST genomic linked microRNAs, i.e. miR335 (SEQ ID NO: 5) and mir335* (SEQ ID NO: 6) are relevant biomarkers of mitochondrial disease. They have notably demonstrated that MEST, miR335 and mir335* are present in cells of patients that have mitochondrial disorders and that the levels MEST, miR335 and miR335* are lower than into corresponding normal cells.

### BRIEF SUMMARY OF THE INVENTION

The invention concerns an *in vitro* method of diagnosis or prognosis of a mitochondrial disease in an individual, which comprises:
a) determining the level of at least one biomarker selected from the group consisting of MEST, microRNA-335, microRNA-335* and a precursor of said microRNA-335 and microRNA-335* in a biological sample from the individual,
b) comparing the level of said at least one biomarker to a control level, and
c) deducing that the individual has developed or is at risk of developing a mitochondrial disease if the level of said at least one biomarker is lower than the control level, or that the individual has not developed or is not at risk of developing a mitochondrial disease if the level of said at least one biomarker is upper than or equal to the control level.

The invention also concerns the use of at least one of MEST, microRNA-335, microRNA-335* and a precursor of said microRNA-335 and microRNA-335* as a biomarker of a mitochondrial disease

Also provided is a ligand that specifically binds to at least one biomarker selected from the group consisting of MEST, microRNA-335, microRNA-335* and a precursor of said microRNA-335 and microRNA-335* for use for diagnosing or prognosing of a mitochondrial disease.

The invention also concerns a pair of primers suitable for amplifying a nucleic acid comprising a sequence selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, SEQ ID NO: 17 a variant, a fragment and a complementary sequence thereof, for use for diagnosing or prognosing of a mitochondrial disease.

The invention also concerns a kit comprising means for detecting the amount and/or expression of at least one nucleic acid comprising a sequence selected from the group consisting of SEQ ID NO: 5, SEQ ID NO: 6, SEQ ID NO: 8, SEQ ID NO: 13, SEQ ID NO: 14, SEQ ID NO: 15, a variant and a fragment thereof, and/or the ligand according to the invention and/or the pair of primers according to the invention.

### DETAILED DESCRIPTION OF THE INVENTION

### Method of diagnosis or prognosis of a mitochondrial disease

The invention concerns an *in vitro* method of diagnosis or prognosis of a mitochondrial disease in an individual, which comprises:
a) determining the level of at least one biomarker selected from the group consisting of MEST, microRNA-335, microRNA-335* and a precursor of said microRNA-335 and microRNA-335* in a biological sample from the individual,
b) comparing the level of said at least one biomarker to a control level, and
c) deducing that the individual has developed or is at risk of developing a mitochondrial disease if the level of said at least one biomarker is lower than the control level, or that the individual has not developed or is not at risk of developing a mitochondrial disease if the level of said at least one biomarker is upper than or equal to the control level.

**"Mitochondrial disease"** refers to disorders to which deficits in mitochondrial respiratory chain activity contribute in the development of pathophysiology of such disorders in a mammal. Mitochondrial disorders may be caused by mutations, acquired or inherited, in mitochondrial DNA (mtDNA) or in nuclear genes that code for mitochondrial components. They may also be the result of acquired mitochondrial dysfunction due to adverse effects of drugs, infections, or other (environmental...) causes.

Congenital mitochondrial diseases are those related to hereditary mutations, deletions, or other defects in mtDNA or in nuclear genes regulating mtDNA integrity, or in nuclear genes encoding proteins that are critical for mitochondrial respiratory chain function. Some of the classical phenotypes of major mitochondrial diseases associated with mutations or deletions of mitochondrial DNA and genomic DNA include Dominant Optic Atrophy (DOA), Leber's Hereditary Optic Neuropathy (LHON), Friedreich ataxia, Type 2 diabetes, aging, Mitochondrial Encephalomyopathy Lactic Acidemia, and Stroke-like episodes (MELAS), Myoclonic Epilepsy with "Ragged Red" (muscle) Fibers (MERRF), Mitochondrial neurogastrointestinal encephalomyopathy (MNGIE), Neurogenic muscle weakness, Ataxia and Retinitis Pigmentosa (NARP), Subacute Necrotizing Encephalomyopathy (Leigh's Syndrome), Progressive External Opthalmoplegia (PEO), Kearns-Sayres Syndrome (PEO, pigmentary retinopathy, ataxia, and heart-block), neurodegenerative diseases such as Alzheimer Disease, Parkinson Disease or Huntington Disease.

Acquired mitochondrial defects comprise primarily 1) damage to mitochondrial DNA due to oxidative processes or aging; 2) mitochondrial dysfunction due to excessive intracellular and intramitochondrial calcium accumulation; 3) inhibition of respiratory chain complexes with endogenous or exogenous respiratory chain inhibitors; 4) acute or chronic oxygen deficiency; and 5) impaired nuclear-mitochondrial interactions, e.g. impaired shuttling of mitochondria in long axons due to microtubule defects, and 6) expression of mitochondrial uncoupling proteins in response to lipids, oxidative damage or inflammation.

The term **"diagnosing"** is used herein to refer to the identification of a molecular or pathological state, disease or condition, such as the identification of a mitochondrial disease or to refer to identification of a patient who has developed or is at risk of developing a mitochondrial disease.

The term **"prognosing"** is used herein to refer to the prediction of the likelihood of developing a mitochondrial disease..

In a preferred embodiment, the method of diagnosis or prognosis of a mitochondrial disease according to the invention comprises the step of determining the level of at least one biomarker selected from the group consisting of MEST, microRNA-335, microRNA-335* and a precursor of said microRNA-335 and microRNA-335* in a biological sample from the individual, said mitochondrial disease may be Friedreich ataxia, Type 2 diabetes, neurodegenerative diseases such as Alzheimer Disease, Parkinson Disease or Huntington Disease, or said mitochondrial disease may be Inherited Optic Neuropathies (IONs) such as LHON or DOA.

In another embodiment, the method of diagnosis or prognosis of a mitochondrial disease according to the invention comprises the step of determining the level of at least MEST protein or MEST gene and the mitochondrial disease is DOA, LHON or Friedreich ataxia.

In another preferred embodiment, method according to the invention comprises the step of determining the level of expression of at least microRNA-335 or a precursor thereof and said mitochondrial disease is LHON or Friedreich ataxia.

In another preferred embodiment, method according to the invention comprises the step of determining the level of expression of at least microRNA-335, microRNA-335* and a precursor of said microRNA-335 and microRNA-335* and said mitochondrial disease is DOA.

**MEST** (MEsoderm-Specific Transcript) gene, also named PEG1 (Paternally Expressed Gene 1) (Gene ID ENSG00000106484) is located on chromosome 7 between positions 130,126,046-130,146,133 (GRCh37: CM000669.1:130126046:130146133:1) and encodes for a member of the [alpha]/[beta] hydrolase fold family. MEST gene has 18 splicing variants among which 12 splicing variants encode for a MEST protein. Five transcripts of MEST gene having sequences SEQ ID NO: 1 (NM_002402.3), SEQ ID NO: 2 (NCBI NM_177524.2), SEQ ID NO: 3 (NCBI NM_177525.2), SEQ ID NO: 11 (NCBI NM_001253900.1) and SEQ ID NO: 12 (NCBI NM_001253901.1) encode for the 4 principally expressed isoforms: MEST isoform 1 (UniProt Q5EB52-1 of sequence SEQ ID NO: 4, NCBI NP_002393.2), MEST isoform 2 or isoform b (UniProt Q5EB52-2, NCBI NP_803490.1, having a sequence identical to amino acids 10 to 335 of SEQ ID NO: 4), MEST 3 or isoform d (UniProt Q5EB52-3, NCBI NP_001240830.1 having a sequence identical to amino acids 10 to 217 and 252 to 335 of SEQ ID NO: 4 (or identical to amino acids 10 to 216 and 251 to 335 of SEQ ID NO: 4, amino acids 217 and 251 being both a Leucine residue) and MEST isoform c (NCBI NP_001240829.1 having a sequence identical to amino acids 1 to 46 and 61 to 335 of SEQ ID NO: 4). MEST proteins having substitutions at residues 90 (E90K), 168 (L168H) or 222 (G222W) in sequence SEQ ID NO: 4 have also been described. MEST transcript or protein includes the transcripts or proteins above mentioned and any other naturally occurring transcript or protein variants or precursors. As way of example, as used herein MEST transcript include notably any nucleic acid comprising residues 282 to 394 (SEQ ID NO: 13), 437 to 905 (SEQ ID NO: 14) or 1008 to 1264 (SEQ ID NO: 15)which are common to the here above mentioned five transcripts of MEST gene or a corresponding sequence in RNA bases. For example, MEST protein further includes proteins comprising amino acids 10 to 46 of SEQ ID NO: 4 (corresponding to sequence SEQ ID NO: 9) and/or 61 to 217 of SEQ ID NO: 4 (corresponding to sequence SEQ ID NO: 10) and/or 252 to 335 of SEQ ID NO: 4 (corresponding to sequence SEQ ID NO: 20) which are common to isoforms 1, 2, 3 and c of MEST protein.

In one embodiment, the level of MEST biomarker is determined by measuring the level of expression of MEST protein and/or gene

According to the invention, the term **"biomarker"** means a distinctive biological or biologically-derived indicator of a process, event, or condition. The term biomarker as used herein refers to a nucleic acid (a gene, a cDNA, a mRNA, a miRNA...) or a protein that is differentially expressed in individuals who has developed or is at risk of developing a mitochondrial disease. In one embodiment, the level of expression of a gene or a nucleic acid is determined by detecting transcription product(s) and/or translation product(s) (ie. protein(s)) of said gene or nucleic acid. The biomarker according to the invention is suitable to be used in methods of diagnosis (e.g. clinical screening), prognosis assessment; identifying a patient who has developed or is at risk of developing mitochondrial disease.

In certain embodiments, a **"gene"** refers to a nucleic acid that is transcribed. In certain aspects, the gene comprises a nucleic acid, and/or encodes a polypeptide. In this respect, the term "gene" is used for simplicity to refer to a nucleic acid comprising a nucleotide sequence that is transcribed, the corresponding sequence in RNA bases and the complement thereof. In particular aspects, the transcribed nucleotide sequence comprises at least one functional protein, polypeptide and/or peptide encoding unit. As will be understood by those in the art, this functional term "gene" includes both genomic sequences, RNA (mRNA, microRNA, Long intergenic non-coding RNAs ...) or cDNA sequences, or smaller engineered nucleic acid segments.

The term **"nucleic acid"** will generally refer to at least one molecule or strand of DNA, RNA, miRNA or a derivative or mimic thereof, comprising at least one nucleobase, such as, for example, a naturally occurring purine or pyrimidine base found in DNA (e.g., adenine "A," guanine "G," thymine "T," and cytosine "C") or RNA (e.g. A, G, uracil "U," and C). The term "nucleic acid" encompasses the terms "oligonucleotide" and "polynucleotide." The term "oligonucleotide" refers to at least one molecule of between about 3 and about 100 nucleobases in length. The term "polynucleotide" refers to at least one molecule of greater than about 100 nucleobases in length. These definitions generally refer to at least one single-stranded molecule, but in specific embodiments will also encompass at least one additional strand that is partially, substantially or fully complementary to the at least one single-stranded molecule. Thus, a nucleic acid may encompass at least one double-stranded molecule that comprises one or more complementary strand(s) or "complement(s)" of a particular sequence comprising a strand of the molecule.

A nucleic acid may be made by any technique known to one of ordinary skill in the art. Non-limiting examples of synthetic nucleic acid, particularly a synthetic oligonucleotide, include a nucleic acid made by *in vitro* chemical synthesis using phosphotriester, phosphite or phosphoramidite chemistry and solid phase techniques such described by Froehler et al., 1986 Nucleic Acids Res. 1986 Jul 11;14(13):5399-407. via deoxynucleoside H-phosphonate intermediates. A non-limiting example of enzymatically produced nucleic acid include one produced by enzymes in amplification reactions such as PCR™ or the synthesis of oligonucleotides. A non-limiting example of a biologically produced nucleic acid includes recombinant nucleic acid production in living cells (see for example, Molecular cloning: a laboratory manual. - 4th ed. / Michael R. Green, Joseph Sambrook. 2012 Cold Spring Harbor, N.Y.). A nucleic acid may be purified on polyacrylamide gels, cesium chloride centrifugation gradients, or by any other means known to one of ordinary skill in the art (see for example, Molecular cloning: a laboratory manual. - 4th ed. / Michael R. Green, Joseph Sambrook. 2012 Cold Spring Harbor, N.Y.). The nucleic acid molecule is preferably isolated, which means that it is essentially free of other nucleic acids. Essentially free from other nucleic acids means that the nucleic acid molecule is at least about 90%, preferably at least about 95% and, more preferably at least about 98% free of other nucleic acids. Preferably, the molecule is essentially pure, which means that the molecule is free not only of other nucleic acids, but also of other materials used in the synthesis and isolation of the molecule. Materials used in synthesis include, for example, enzymes. Materials used in isolation include, for example, gels, such as SDS-PAGE. The molecule is at least about 90% free, preferably at least about 95% free and, more preferably at least about 98% free of other nucleic acids and such other materials.

**"MicroRNAs"** or **"miRNAs"** are endogenously encoded RNAs that are about 22-nucleotide-long, that post-transcriptionally regulate target genes and are generally expressed in a highly tissue-specific or developmental-stage-specific fashion. One can design and express artificial miRNAs based on the features of existing miRNA genes. microRNA-335 (miR-335 or hsa-miR-335-5p, miRbase (http://www.mirbase.org) réf: MIMAT0000765, SEQ ID NO: 5) and microRNA-335* (miR-335* or hsa-miR-335-3p MIMAT0004703, SEQ ID NO: 6) and their precursor pre-miR-335 (hsa-mir-335MI0000816, SEQ ID NO: 7) are MEST genomic linked microRNAs.

A **"precursor"** according to the invention may be a protein precursor (such as a molecule containing a protein sequence of the invention) or a RNA precursor (such as a molecule containing a RNA sequence of the invention). A **"miRNA precursor"** or **"miRNA precursor sequence"** means a transcript that originates from a genomic DNA and that comprises a non-coding, structured RNA comprising one or more miRNA sequences. For example, in certain embodiments a miRNA precursor is a pre-miRNA such as pri-miR-335 or for example pre-miR-335 (hsa-mir-335MI0000816, SEQ ID NO: 7) which is the precursor of microRNA-335* and microRNA-335.

According to the invention, a **level of at least one biomarker** of **the invention lower than the control level is indicative that the individual has developed or is at risk of developing a mitochondrial disease.** Indeed, according to the method of the invention, detection of a level of MEST and microRNA-335 lower than their respective control levels is indicative that the individual has developed or is at risk of developing a mitochondrial disease. Moreover, according to the method of the invention, detection of a level of MEST and microRNA-335* lower than their respective control levels, or, detection of a level of microRNA-335 and microRNA-335* lower than their respective control levels, or detection of a level of MEST, microRNA-335 and microRNA-335* lower than their respective control levels or, detection of a level of MEST and a microRNA-335 precursor lower than their respective control levels or, detection of a level of MEST and a microRNA-335* precursor lower than their respective control levels or, detection of a level of MEST, a microRNA-335 precursor and a microRNA-335* precursor lower than their respective control levels or, detection of a level of MEST, a microRNA-335 and a microRNA-335* precursor lower than their respective control levels or, detection of a level of MEST, a microRNA-335 precursor and a microRNA-335* lower than their respective control levels is indicative that the individual has developed or is at risk of developing a mitochondrial disease.

The term **"the level of a biomarker is lower than the control level"** as used herein, means that there is a statistically significant reduction between the level of said biomarker as measured in the biological sample and the control level. In one embodiment, the level of biomarker can be compared to the control level using the ratio of the level of said biomarker as compared with the control level or using p-value.

According to the invention, a level of a biomarker of the invention upper than or equal to the control level, is indicative that the individual has not developed or is not at risk of developing a mitochondrial disease. Indeed, according to the method of the invention, detection of a level of MEST and microRNA-335 upper than or equal to their respective control levels, is indicative that the individual has not developed or is not at risk of developing a mitochondrial disease. Moreover, according to the method of the invention, detection of a level of MEST and microRNA-335* upper than or equal to their respective control levels, or, detection of a level of microRNA-335 and microRNA-335* upper than or equal to their respective control levels, or detection of a level of MEST, microRNA-335 and microRNA-335* upper than or equal to their respective control levels, or, detection of a level of MEST and a microRNA-335 precursor upper than or equal to their respective control levels or, detection of a level of MEST and a microRNA-335* precursor upper than or equal to their respective control levels or, detection of a level of MEST, a microRNA-335 precursor and a microRNA-335* precursor upper than or equal to their respective control levels or, detection of a level of MEST, a microRNA-335 precursor and a microRNA-335* upper than or equal to their respective control levels or, detection of a level of MEST, a microRNA-335 and a microRNA-335* precursor upper than or equal to their respective control levels is indicative that the individual has not developed or is not at risk of developing a mitochondrial disease.

The term **"the level of a biomarker is upper than the control level"** as used herein means that there is a statistically significant increase between the level of said biomarker as measured in the biological sample and the control level. In one embodiment, the level of biomarker can be compared to the control level using the ratio of the level of said biomarker as compared with the control level or using p-value.

The term **"the level of a biomarker is equal to the control level"** as used herein means that there is no or little difference in the level of said biomarker between the level measured in the biological sample and the control level. There is a similar level of said biomarker between two samples when there is no statistically significant difference in the level of said biomarker and the control level.

According to the invention, a **"control level"** is the control level for one specific biomarker according to the invention and may be obtained from a biological sample from a healthy individual or a group of healthy individuals. The control level can be any number of statistical measures to distinguish between a level indicative that an individual has developed or is at risk of developing a mitochondrial disease and a level indicative that an individual has not developed or is not at risk of developing a mitochondrial disease, including Mean and Median expression levels, and/or cut-off or threshold gene expression or fold change values as determined in an individual or a group of individuals.

A **"biological sample"** encompasses a variety of sample types obtained from an individual and can be used in a diagnostic or monitoring assay. The definition encompasses a clinical sample, solid tissue samples such as a biopsy specimen or tissue cultures, cells in culture, cell supernatants, cell lysates or cells derived therefrom and the progeny thereof, and also includes biological fluid such as serum, plasma, blood and other liquid samples of biological origin. Preferably, the tissue is muscle or skin. More preferably, the biological sample comprises muscle cells or skin fibroblasts. The method of the invention is particularly relevant in that fibroblasts are easily accessible patient biological samples for biomarkers and diagnostic assays. The definition of biological sample also includes samples that have been manipulated in any way after their procurement, such as by treatment with reagents, solubilization, purification or enrichment for certain components, such as polypeptides or nucleic acids.

When the control level is obtained from a biological sample from **"a healthy individual" "a group of healthy individuals"** also named "control sample", the expression "healthy individual(s)" refers an individual or a reference group of individuals who are not suffering from or who did not develop a mitochondrial disease.

According to the invention, the level of expression of MEST gene is determined by detecting a nucleic acid comprising SEQ ID NO: 8, SEQ ID NO: 13, SEQ ID NO: 14 or SEQ ID NO: 15 a variant, a fragment, a complementary sequence or a corresponding RNA sequence thereof, preferably, a nucleic acid comprising SEQ ID NO: 1, SEQ ID NO: 2, SEQ ID NO: 3, SEQ ID NO: 11 or SEQ ID NO: 12, a variant, a fragment, a complementary sequence or a corresponding RNA sequence thereof.

Level of expression of a gene or a nucleic acid can be performed by methods which are well known to the person skilled in the art, including in particular direct hybridization based assays and amplification-based assays.

The methods using direct hybridization based assays refer to pairing and binding of a nucleotide sequence (probe) to a complementary sequence to MEST RNA and cDNA. The probe is designed using partial or full MEST nucleotide sequence. The quantification of MEST transcript expression utilizes methods well known in the art such as nucleic acid arrays, RNase protection assays, Northern-Blots, Slot-Blots and other technologies. The resulting complexes from hybridization are quantified by the nucleotide probes by well known technologies in the art such as fluorescence, luminescence, radioelement labelling and other technologies.

The methods using amplification-based assays refer to technologies amplifying a specific transcript such as MEST transcript (precursor/mRNA/cDNA) using methods, well known in the art, such as Polymerase Chain Reaction (PCR). The PCR technology uses, among others components, specific primers (direct and forward) designed using the nucleotide sequences of the MEST transcript to amplify partially or fully MEST nucleotide sequence. Quantification uses nucleotide probes by well known technologies in the art such as fluorescence, luminescence, radioelement labelling and other technologies. A representative technology is the TaqMan technique developed by, among other companies, Applied Biosystems (Perkin Elmer) in which a specific transcript as MEST is quantified by the release of a fluorescent reporter dye. The fluorescent reporter dye is release from a specific hybridisation probe in real-time during a polymerase chain reaction (PCR) and is proportional to the accumulation of the PCR product.

Level of expression of a gene or a nucleic acid may further be assessed by using immunologic methods such as antibodies directed against nucleic acids or antibodies directed against miRNAs.

According to the invention, the level of the biomarker microRNA-335 is determined by detecting a nucleic acid comprising SEQ ID NO: 5, a variant and a fragment thereof. Preferably a sequence comprising SEQ ID NO: 5 is its precursor of sequence SEQ ID NO: 7.

According to the invention, the level of the biomarker microRNA-335* is determined by detecting a nucleic acid comprising SEQ ID NO: 6, a variant or a fragment thereof. Preferably a sequence comprising SEQ ID NO: 6 is its precursor of sequence SEQ ID NO: 7.

The quantification of microRNAs, such as mir335 and mir335*, uses similar techniques as can be used to quantify MEST transcript, with some experimental adaptation, well known in the art, due to the short sequences of the microRNAs. For example, the reverse transcription of microRNA to cDNA is realized using hairloop primers such as developed by the Applied Biosystems company (Perkin Elmer).

In one embodiment, the method of the invention comprises the step of determining the level of expression of at least one, 2, 3, 4, or 6 different nucleic acid sequences wherein each sequence comprises a sequence selected from the group consisting of SEQ ID NO: 5, SEQ ID NO: 6, SEQ ID NO: 8, SEQ ID NO: 13, SEQ ID NO: 14, SEQ ID NO: 15, a corresponding RNA sequence of SEQ ID NO: 8, SEQ ID NO: 13, SEQ ID NO: 14 or SEQ ID NO: 15, a variant, a fragment or a complementary sequence thereof. Notably, the method of the invention comprises the step of determining the level of expression of at least one 2 different nucleic acid sequences wherein each sequence comprises a sequence selected from the group consisting of SEQ ID NO: 5 and SEQ ID NO: 6 or SEQ ID NO: 6 and SEQ ID NO: 8 or SEQ ID NO: 5 and SEQ ID NO: 8, SEQ ID NO: 13 and SEQ ID NO: 5 or SEQ ID NO: 14 and SEQ ID NO: 5 or SEQ ID NO: 13 and SEQ ID NO: 6 or SEQ ID NO: 14 and SEQ ID NO: 6 or SEQ ID NO: 13 and SEQ ID NO: 8 or SEQ ID NO: 14 and SEQ ID NO: 8, or SEQ ID NO: 15 and SEQ ID NO: 6 or SEQ ID NO: 15 and SEQ ID NO: 8, or SEQ ID NO: 15 and SEQ ID NO: 13 or SEQ ID NO: 15 and SEQ ID NO: 14, a variant, a fragment, a complementary sequence or a corresponding RNA sequence thereof. Typically, the method of the invention comprises the step of determining the level of expression of at least one 3 different nucleic acid sequences wherein each sequence comprises a sequence selected from the group consisting of SEQ ID NO: 5, SEQ ID NO: 6 and SEQ ID NO: 8 or SEQ ID NO: 5, SEQ ID NO: 6 and SEQ ID NO: 13 or SEQ ID NO: 5, SEQ ID NO: 6 and SEQ ID NO: 14 or SEQ ID NO: 5, SEQ ID NO: 6 and SEQ ID NO: 15 or SEQ ID NO: 5, SEQ ID NO: 8 and SEQ ID NO: 13 or SEQ ID NO: 5, SEQ ID NO: 8 and SEQ ID NO: 14 or SEQ ID NO: 5, SEQ ID NO: 8 and SEQ ID NO: 15 or SEQ ID NO: 5, SEQ ID NO: 13 and SEQ ID NO: 14 or SEQ ID NO: 5, SEQ ID NO: 13 and SEQ ID NO: 15 or SEQ ID NO: 5, SEQ ID NO: 14 and SEQ ID NO: 15 a variant, a fragment, a complementary sequence or a corresponding RNA sequence thereof. Notably, the method of the invention comprises the step of determining the level of expression of at least one 4 different nucleic acid sequences wherein each sequence comprises a sequence selected from the group consisting of SEQ ID NO: 5, SEQ ID NO: 6, SEQ ID NO: 8 and SEQ ID NO: 13 or SEQ ID NO: 5, SEQ ID NO: 6, SEQ ID NO: 8 and SEQ ID NO: 14 or SEQ ID NO: 5, SEQ ID NO: 6, SEQ ID NO: 8 and SEQ ID NO: 15 or SEQ ID NO: 6, SEQ ID NO: 8, SEQ ID NO: 13 and SEQ ID NO: 14 or SEQ ID NO: 6, SEQ ID NO: 8, SEQ ID NO: 13 and SEQ ID NO: 15 or SEQ ID NO: 8, SEQ ID NO: 13, SEQ ID NO: 14 and SEQ ID NO: 15 a variant, a fragment, a complementary sequence or a corresponding RNA sequence thereof. Typically, the method of the invention comprises the step of determining the level of expression of at least one 5 different nucleic acid sequences wherein each sequence comprises a sequence selected from the group consisting of SEQ ID NO: 5, SEQ ID NO: 6, SEQ ID NO: 8, SEQ ID NO: 13 and SEQ ID NO: 14 or SEQ ID NO: 5, SEQ ID NO: 6, SEQ ID NO: 8, SEQ ID NO: 13 and SEQ ID NO: 15 or SEQ ID NO: 5, SEQ ID NO: 6, SEQ ID NO: 13, SEQ ID NO: 14 and SEQ ID NO: 15 or SEQ ID NO: 5, SEQ ID NO: 8, SEQ ID NO: 13, SEQ ID NO: 14 and SEQ ID NO: 15 or SEQ ID NO: 5, SEQ ID NO: 6, SEQ ID NO: 8, SEQ ID NO: 14 and SEQ ID NO: 15 or SEQ ID NO: 6, SEQ ID NO: 8, SEQ ID NO: 13, SEQ ID NO: 14 and SEQ ID NO: 15, or a variant, a fragment, a complementary sequence or a corresponding RNA sequence thereof. The method of the invention may comprise the step of determining the level of expression of at least 2, 3, 4, 5, 6, 7, 8, 9, 10, 11 or 12 different nucleic acid sequences wherein each sequence comprises a sequence selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, a variant, a fragment, a complementary sequence or a corresponding RNA sequence thereof

Preferably, the level of expression of MEST protein is determined by detecting a polypeptide comprising SEQ ID NO: 9 and/or SEQ ID NO: 10, and/or SEQ ID NO: 20, a variant or a fragment thereof. More preferably, the level of expression of MEST protein is determined by detecting a polypeptide comprising the sequence SEQ ID NO: 4, a variant or a fragment thereof.

As used herein the term **"protein"** or **"polypeptide"** refers to any chain of amino acids linked by peptide bonds, regardless of length or post-translational modification. Polypeptides include natural proteins, synthetic or recombinant polypeptides and peptides (i.e. polypeptides of less than 50 amino acids) as well as hybrid, post-translationally modified polypeptides, and peptidomimetic.

Level of expression of a protein or a polypeptide may be assessed by using immunologic methods such as detection using polyclonal or monoclonal antibodies, chimeric antibody or humanized antibodies. The level of expression of MEST protein is quantified using technologies well known by the art.

Suitable immunologic methods include enzyme linked immunoassays (ELISA), sandwich, direct, indirect, or competitive ELISA assays, enzyme linked immunospotassays (ELIspot), radio immunoassays (RIA), flow-cytometry assays (FACS), immunohistochemistry, Western Blot, fluorescence resonance energy transfer (FRET) assays, protein chip assays using for example antibodies, antibody fragments, receptor ligands or other agents binding the proteins coded by the sequence such as for example MEST gene.

As used herein, the term **"amino acid"** refers to the 20 standard alpha-amino acids as well as naturally occurring and synthetic derivatives. A polypeptide may contain L or D amino acids or a combination thereof.

The term **"variants"** includes protein and nucleic acid variants. Variant proteins may be naturally occurring variants, such as splice variants, alleles and isoforms. Variations in amino acid sequence may be introduced by substitution, deletion or insertion of one or more codons into the nucleic acid sequence encoding the protein that results in a change in the amino acid sequence of the protein. For example, a variant of SEQ ID NO: 4 may be a protein comprising a sequence identical to amino acids 10 to 335 of SEQ ID NO: 4 or a protein comprising a sequence identical to amino acids 10 to 217 and/or 252 to 335 of SEQ ID NO: 4 such as isoforms 2 and 3 of MEST protein. Variant proteins may be a protein having a conservative or non-conservative substitution. For example, a variant of SEQ ID NO: 4 may be a polypeptide having at least one substitution at residues E90 in sequence SEQ ID NO: 4 such as E90K, L168 in sequence SEQ ID NO: 4 such as L168H or at residue G222 in sequence SEQ ID NO: 4 such as G222W. Variant proteins may include proteins that have at least about 80% amino acid sequence identity with a polypeptide sequence disclosed herein. Preferably, a variant protein will have at least about 80%, 81%, 82%, 83%, 84%, 85%, 86%, 87%, 88%, 89%, 90%, 91%, 92%, 93%, 94%, 95%, 96%, 97%, 98%, 99% amino acid sequence identity to a full-length polypeptide sequence or a fragment of a polypeptide sequence as disclosed herein. For example, a variant of SEQ ID NO: 4 may be a polypeptide having at least 87% of identity with the sequence SEQ ID NO: 4 such as the MEST isoform 2 (UniProt Ref: Q5EB52-2) or MEST isoform 3 (UniProt Ref: Q5EB52-3). Amino acid sequence identity is defined as the percentage of amino acid residues in the variant sequence that are identical with the amino acid residues in the reference sequence, after aligning the sequences and introducing gaps, if necessary, to achieve the maximum percent sequence identity, and not considering any conservative substitutions as part of the sequence identity. Sequence identity may be determined over the full length of the variant sequence, the full length of the reference sequence, or both. The percentage of identity for protein sequences may be calculated by performing a pairwise global alignment based on the Needleman-Wunsch alignment algorithm to find the optimum alignment (including gaps) of two sequences along their entire length, for instance using Needle, and using the BLOSUM62 matrix with a gap opening penalty of 10 and a gap extension penalty of 0.5.

Variant nucleic acid sequences may include nucleic acid sequences that have at least about 80% nucleic acid sequence identity with a nucleic acid sequence disclosed herein. Preferably, a variant nucleic acid sequence will have at least about 80%, 81%, 82%, 83%, 84%, 85%, 86%, 87%, 88%, 89%, 90%, 91%, 92%, 93%, 94%, 95%, 96%, 97%, 98%, 99% nucleic acid sequence identity to a full-length nucleic acid sequence or a fragment of a nucleic acid sequence as disclosed herein. Examples of nucleic acid sequences having at least 92% of identity with the sequence SEQ ID NO: 1 is the nucleic acids SEQ ID NO: 2, SEQ ID NO: 3, SEQ ID NO: 11 and SEQ ID NO: 12. A variant of SEQ ID NO: 1 may be a nucleic acid sequence which has been described as a transcript of MEST gene such as for example SEQ ID NO: 2, SEQ ID NO: 3, SEQ ID NO: 11, or 12. Nucleic acid sequence identity can be calculated by methods well-known to one of skill in the art. The percentage of identity may be calculated by performing a pairwise global alignment based on the Needleman-Wunsch alignment algorithm to find the optimum alignment (including gaps) of two sequences along their entire length, for instance using Needle, and using the DNAFULL matrix with a gap opening penalty of 10 and a gap extension penalty of 0.5.

The term **"fragments"** includes protein and nucleic acid fragments. A protein sequence may be truncated at the N-terminus or C-terminus, or may lack internal residues, for example, when compared with a full length protein. Certain fragments lack amino acid residues that are not essential for hydrolase fold activity of MEST protein. Preferably, said fragments are at least about 10, 20, 30, 40, 50, 60, 70, 80, 90, 100, 110, 120, 150, 250, 300 or more amino acids in length. Fragments of the polypeptide of sequence SEQ ID NO: 4 of the invention are for example, residues 10-46 of SEQ ID NO: 4 (corresponding to SEQ ID NO: 9), and/or 61 to 217 of SEQ ID NO: 4 (corresponding to sequence SEQ ID NO: 10) and/or residues 252-335 of SEQ ID NO: 4 (corresponding to SEQ ID NO: 20), and/or residues 1-217 or 61-335 of SEQ ID NO: 4.

A nucleic acid sequence may be truncated at the 5'-terminus or 3'-terminus, or may lack internal residues, for example, when compared with MEST gene. Certain fragments lack residues that are not essential for enzymatic activity of the encoded protein. Preferably, said fragments are at least about 10, 15, 20, 30, 40, 100, 200, 300, 500, 1000, 2000 or more nucleotides in length. A fragment of the nucleic acid sequence SEQ ID NO: 1 of the invention is for example, residues 282 to 2513 of sequence SEQ ID NO: 1 which is common to all the transcripts SEQ ID NO: 1, SEQ ID NO: 2 and SEQ ID NO: 3 of MEST gene. Other fragments of the nucleic acid sequence SEQ ID NO: 1 of the invention are for example, residues 282 to 394 (SEQ ID NO: 13), 437 to 905 (SEQ ID NO: 14), 1008 to 2513 of sequence SEQ ID NO: 1 which are common to all the transcripts SEQ ID NO: 1, SEQ ID NO: 2, SEQ ID NO: 3, SEQ ID NO: 11 and SEQ ID NO: 12. The end of coding sequence of SEQ ID NO: 1 being at residue 1264, the fragment 282 to 2513 may be reduced to 282 to 1264 (corresponding to sequence SEQ ID NO: 8) and the fragment 1008 to 2513 may be reduced to 1008 to 1264 (SEQ ID NO: 15).

In one embodiment, the method of the invention comprises the step of determining the level of expression of at least 2, 3, 4, 5, 6, 7, 8 or 9 different sequences wherein each sequence comprises a sequence selected from the group consisting of SEQ ID NO: 5, SEQ ID NO: 6, SEQ ID NO: 8, SEQ ID NO: 9, SEQ ID NO: 10, SEQ ID NO: 13, SEQ ID NO: 14, SEQ ID NO: 15, SEQ ID NO: 20, a variant, a fragment thereof.Typically, the method of the invention comprises the step of determining the level of expression of at least one 2 different sequences wherein each sequence comprises a sequence selected from the group consisting of SEQ ID NO: 5 and SEQ ID NO: 6 or, SEQ ID NO: 5 and SEQ ID NO: 8 or, SEQ ID NO: 5 and SEQ ID NO: 9 or, SEQ ID NO: 5 and SEQ ID NO: 10 or, SEQ ID NO: 5 and SEQ ID NO: 13 or, SEQ ID NO: 5 and SEQ ID NO: 14 or, SEQ ID NO: 5 and SEQ ID NO: 15 or, SEQ ID NO: 5 and SEQ ID NO: 20 or, SEQ ID NO: 6 and SEQ ID NO: 8 or, SEQ ID NO: 6 and SEQ ID NO: 9 or, SEQ ID NO: 6 and SEQ ID NO: 10 or, SEQ ID NO: 6 and SEQ ID NO: 13 or, SEQ ID NO: 6 and SEQ ID NO: 14 or, SEQ ID NO: 6 and SEQ ID NO: 15 or, SEQ ID NO: 6 and SEQ ID NO: 20 or, SEQ ID NO: 8 and SEQ ID NO: 9 or, SEQ ID NO: 8 and SEQ ID NO: 10 or, SEQ ID NO: 8 and SEQ ID NO: 13 or, SEQ ID NO: 8 and SEQ ID NO: 14 or, SEQ ID NO: 8 and SEQ ID NO: 15 or, SEQ ID NO: 8 and SEQ ID NO: 20 or, SEQ ID NO: 9 and SEQ ID NO: 10 or, SEQ ID NO: 9 and SEQ ID NO: 13 or, SEQ ID NO: 9 and SEQ ID NO: 14 or, SEQ ID NO: 9 and SEQ ID NO: 15 or, SEQ ID NO: 9 and SEQ ID NO: 20 or, SEQ ID NO: 10 and SEQ ID NO: 13 or, SEQ ID NO: 10 and SEQ ID NO: 14 or, SEQ ID NO: 10 and SEQ ID NO: 15 or, SEQ ID NO: 10 and SEQ ID NO: 20 or, SEQ ID NO: 13 and SEQ ID NO: 14 or, SEQ ID NO: 13 and SEQ ID NO: 15 or, SEQ ID NO: 13 and SEQ ID NO: 20 or, SEQ ID NO: 14 and SEQ ID NO: 15 or, SEQ ID NO: 14 and SEQ ID NO: 20 or, SEQ ID NO: 15 and SEQ ID NO: 20 a variant, a fragment thereof. Notably, the method of the invention comprises the step of determining the level of expression of at least one 3 different sequences wherein each sequence comprises a sequence selected from the group consisting of SEQ ID NO: 5, SEQ ID NO: 6 and SEQ ID NO: 8 or SEQ ID NO: 5, SEQ ID NO: 6 and SEQ ID NO: 9 or SEQ ID NO: 5, SEQ ID NO: 6 and SEQ ID NO: 10 or SEQ ID NO: 5, SEQ ID NO: 6 and SEQ ID NO: 13 or SEQ ID NO: 5, SEQ ID NO: 6 and SEQ ID NO: 14 or SEQ ID NO: 5, SEQ ID NO: 6 and SEQ ID NO: 15 or SEQ ID NO: 5, SEQ ID NO: 6 and SEQ ID NO: 20 or SEQ ID NO: 6, SEQ ID NO: 8 and SEQ ID NO: 9 or SEQ ID NO: 6, SEQ ID NO: 8 and SEQ ID NO: 10 or SEQ ID NO: 6, SEQ ID NO: 8 and SEQ ID NO: 13 or SEQ ID NO: 6, SEQ ID NO: 8 and SEQ ID NO: 14 or SEQ ID NO: 6, SEQ ID NO: 8 and SEQ ID NO: 15 or SEQ ID NO: 6, SEQ ID NO: 8 and SEQ ID NO: 20 or SEQ ID NO: 8-10 or SEQ ID NO: 8, SEQ ID NO: 9 and SEQ ID NO: 13 or SEQ ID NO: 8, SEQ ID NO: 9 and SEQ ID NO: 14 or SEQ ID NO: 8, SEQ ID NO: 9 and SEQ ID NO: 15 or SEQ ID NO: 8, SEQ ID NO: 13 and SEQ ID NO: 14 or SEQ ID NO: 8, SEQ ID NO: 14 and SEQ ID NO: 15 or SEQ ID NOs: 13-15 or SEQ ID NO: 14, SEQ ID NO: 15 and SEQ ID NO: 20 or SEQ ID NO: 9, SEQ ID NO: 10 and SEQ ID NO: 20 a variant, a fragment thereof. Preferably, the method of the invention comprises the step of determining the level of expression of at least one 4 different sequences wherein each sequence comprises a sequence selected from the group consisting of SEQ ID NO: 5, SEQ ID NO: 6, SEQ ID NO: 8 and SEQ ID NO: 13 or SEQ ID NO: 5, SEQ ID NO: 6, SEQ ID NO: 8 and SEQ ID NO: 14 or SEQ ID NO: 5, SEQ ID NO: 6, SEQ ID NO: 8 and SEQ ID NO: 15 or SEQ ID NO: 6, SEQ ID NO: 8, SEQ ID NO: 13 and SEQ ID NO: 14 or SEQ ID NO: 6, SEQ ID NO: 8, SEQ ID NO: 13 and SEQ ID NO: 15 or SEQ ID NO: 8, SEQ ID NO: 13, SEQ ID NO: 14 and SEQ ID NO: 15 or SEQ ID NO: 6, SEQ ID NO: 9, SEQ ID NO: 10 and SEQ ID NO: 13 or SEQ ID NO: 6, SEQ ID NO: 9, SEQ ID NO: 10 and SEQ ID NO: 14 or SEQ ID NO: 6, SEQ ID NO: 9, SEQ ID NO: 10 and SEQ ID NO: 15 or SEQ ID NO: 6, SEQ ID NO: 9, SEQ ID NO: 10 and SEQ ID NO: 20 or SEQ ID NO: 5, SEQ ID NO: 9, SEQ ID NO: 10 and SEQ ID NO: 13 or SEQ ID NO: 5, SEQ ID NO: 9, SEQ ID NO: 10 and SEQ ID NO: 14 or SEQ ID NO: 5, SEQ ID NO: 9, SEQ ID NO: 10 and SEQ ID NO: 15 or SEQ ID NO: 5, SEQ ID NO: 9, SEQ ID NO: 10 and SEQ ID NO: 20 a variant, a fragment thereof. Preferably, the method of the invention comprises the step of determining the level of expression of at least one 5 different sequences wherein each sequence comprises a sequence selected from the group consisting of SEQ ID NO: 5, SEQ ID NO: 6, SEQ ID NO: 8, SEQ ID NO: 13 and SEQ ID NO: 14 or SEQ ID NO: 5, SEQ ID NO: 6, SEQ ID NO: 8, SEQ ID NO: 13 and SEQ ID NO: 15 or SEQ ID NO: 5, SEQ ID NO: 6, SEQ ID NO: 13, SEQ ID NO: 14 and SEQ ID NO: 15 or SEQ ID NO: 5, SEQ ID NO: 8, SEQ ID NO: 13, SEQ ID NO: 14 and SEQ ID NO: 15 or SEQ ID NO: 5, SEQ ID NO: 6, SEQ ID NO: 8, SEQ ID NO: 14 and SEQ ID NO: 15 or SEQ ID NO: 6, SEQ ID NO: 8, SEQ ID NO: 13, SEQ ID NO: 14 and SEQ ID NO: 15, or SEQ ID NO: 5, SEQ ID NO: 6, SEQ ID NO: 8, SEQ ID NO: 9 and SEQ ID NO: 13 or SEQ ID NO: 5, SEQ ID NO: 6, SEQ ID NO: 8, SEQ ID NO: 9 and SEQ ID NO: 14 or SEQ ID NO: 5, SEQ ID NO: 6, SEQ ID NO: 8, SEQ ID NO: 9 and SEQ ID NO: 15 or SEQ ID NO: 5, SEQ ID NO: 6, SEQ ID NO: 8, SEQ ID NO: 9 and SEQ ID NO: 20 or SEQ ID NO: 5, SEQ ID NO: 6, SEQ ID NO: 13, SEQ ID NO: 9 and SEQ ID NO: 13 or SEQ ID NO: 5, SEQ ID NO: 6, SEQ ID NO: 13, SEQ ID NO: 9 and SEQ ID NO: 14 or SEQ ID NO: 5, SEQ ID NO: 6, SEQ ID NO: 13, SEQ ID NO: 9 and SEQ ID NO: 15 or SEQ ID NO: 5, SEQ ID NO: 6, SEQ ID NO: 13, SEQ ID NO: 9 and SEQ ID NO: 20 or SEQ ID NO: 5, SEQ ID NO: 6, SEQ ID NO: 8, SEQ ID NO: 10 and SEQ ID NO: 13 or SEQ ID NO: 5, SEQ ID NO: 6, SEQ ID NO: 8, SEQ ID NO: 10 and SEQ ID NO: 14 or SEQ ID NO: 5, SEQ ID NO: 6, SEQ ID NO: 8, SEQ ID NO: 10 and SEQ ID NO: 15 or SEQ ID NO: 5, SEQ ID NO: 6, SEQ ID NO: 8, SEQ ID NO: 10 and SEQ ID NO: 20 or SEQ ID NO: 5, SEQ ID NO: 6, SEQ ID NO: 13, SEQ ID NO: 10 and SEQ ID NO: 13 or SEQ ID NO: 5, SEQ ID NO: 6, SEQ ID NO: 13, SEQ ID NO: 10 and SEQ ID NO: 14 or SEQ ID NO: 5, SEQ ID NO: 6, SEQ ID NO: 13, SEQ ID NO: 10 and SEQ ID NO: 15 or SEQ ID NO: 5, SEQ ID NO: 6, SEQ ID NO: 13, SEQ ID NO: 10 and SEQ ID NO: 20 or SEQ ID NO: 5, SEQ ID NO: 6, SEQ ID NO: 9, SEQ ID NO: 10 and SEQ ID NO: 13 or SEQ ID NO: 5, SEQ ID NO: 6, SEQ ID NO: 9, SEQ ID NO: 10 and SEQ ID NO: 14 or SEQ ID NO: 5, SEQ ID NO: 6, SEQ ID NO: 9, SEQ ID NO: 10 and SEQ ID NO: 15 or SEQ ID NO: 5, SEQ ID NO: 6, SEQ ID NO: 9, SEQ ID NO: 10 and SEQ ID NO: 20 a variant, a fragment thereof

### The use of the sequences of the invention as biomarkers of a mitochondrial disease

The invention also concerns the use of at least one of MEST, microRNA-335, microRNA-335*, a precursor of said microRNA-335 and microRNA-335*, a variant or a fragment thereof as a biomarker of a mitochondrial disease.

Preferably, said biomarker comprises a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 20 a variant and a fragment thereof.

In one embodiment, the level of expression of said biomarker is compared to a control level.

### Primers, ligands and Kits containing thereof for use for diagnosing or prognosing of a mitochondrial disease

Also provided is a ligand specifically binding to a biomarker selected from the group consisting of MEST, microRNA-335, microRNA-335*, a precursor of said microRNA-335 and microRNA-335*, a variant and a fragment thereof, for use for diagnosing or prognosing of a mitochondrial disease.

Preferably, said biomarker comprises a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 20 ,a variant and a fragment thereof.

The term **"ligand"** refers to an entity that specifically binds to the biomarker. The ligand may be a nucleic acid such as a probe or a primer or may be a polypeptide such as an antibody.

According to one embodiment, said ligand is a probe specifically hybridizes to a biomarker comprising a nucleic acid selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15 a variant, a fragment and a complementary sequence thereof.

The term **"probe"** refers to an isolated nucleic acid capable of hybridizing to a target nucleic acid. A detectable label or reporter molecule can be attached to a probe. Typical labels include radioactive isotopes, enzyme substrates, co-factors, ligands, chemiluminescent or fluorescent agents, haptens, and enzymes. Methods for labeling and guidance in the choice of labels appropriate for various purposes are discussed, for example, in Sambrook et al. ((1989) Molecular Cloning; A Laboratory Manual, Cold Spring Harbor*).* Probes are preferably at least 12, 15, 20, 25, 30 nucleotide long. Probes may be less than 200, 150, 100, or preferably 50 nucleotide long.

Probes can be used for diagnosing or prognosing mitochondrial disease by identifying that at least one sequence selected from the group consisting of SEQ ID NO: 1, SEQ ID NO: 2, SEQ ID NO: 3, SEQ ID NO: 5, SEQ ID NO: 6 SEQ ID NO: 7, SEQ ID NO: 8, SEQ ID NO: 11, SEQ ID NO: 12, SEQ ID NO: 13, SEQ ID NO: 14 and SEQ ID NO: 15 is under-expressed in a biological sample comparing with a control sample or a control level. Contacting a total RNA sample (transcriptome) of a biological sample, with the probe, under conditions which allow hybridization of the probe with its corresponding fragment in the nucleic acid, results in the formation of a nucleic acid/probe hybrid. The formation of this hybrid can be detected (e.g., via labeling of the nucleic acid or probe), whereby the formation of this hybrid indicates the expression of the corresponding sequence. Such identification methods based on hybridization with a specific probe (either on a solid phase carrier or in solution) have been described in the art. The specific probe is preferably a sequence which, under optimized conditions, hybridizes specifically to SEQ ID NO: 1, SEQ ID NO: 2, SEQ ID NO: 3, SEQ ID NO: 5, SEQ ID NO: 6 SEQ ID NO: 7, SEQ ID NO: 8, SEQ ID NO: 11, SEQ ID NO: 12, SEQ ID NO: 13, SEQ ID NO: 14 or SEQ ID NO: 15.

Preferably, the probe comprises a sequence of between 50 and 800 bp, preferably of 60 and 500 bp or 100 to 350 bp which is at least 80%, preferably between 80 and 85%, more preferably between 85 and 90%, especially preferably between 90 and 95%, most preferably between 95% and 100% identical (or complementary) to the nucleotide sequence of a specific region. Preferably, the probe will comprise a sequence of about 15 to about 100, or 25 to about 80 and notably, 15, 20, 30, 40, 50, 60, 70, 80, 90, 100, 115, 120, 200, 250, 300, 350, 800 contiguous nucleotides identical (or complementary) to SEQ ID NO: 1, SEQ ID NO: 2, SEQ ID NO: 3, SEQ ID NO: 5, SEQ ID NO: 6 SEQ ID NO: 7, SEQ ID NO: 8, SEQ ID NO: 11, SEQ ID NO: 12, SEQ ID NO: 13, SEQ ID NO: 14 or SEQ ID NO: 15.

A probe according to the invention may comprise, consist or consist essentially of a nucleotide sequence SEQ ID NO: 16 or SEQ ID NO: 18 or may comprise, consist or consist essentially of a nucleotide sequence within or on the vicinity of sequence SEQ ID NO: 16 or SEQ ID NO: 18.

Probes may also comprise sequence SEQ ID NO: 16 or SEQ ID NO: 18 located at their extreme 3' end or their extreme 5' end, and further comprise unrelated sequences or sequences derived from the mentioned nucleotide sequences, but comprising mismatches.

Examples of MEST probes are commercialized by Applied Biosystems™ (MEST(mouse) Cat. # 4331182 Mm00484993_m1; MEST (human) Cat. # 4331182 Hs00853380_g1). An example of miR-335 probe is commercialized by Applied Biosystems™ (Ref Cat. # 4427975 Mm-miR-335). An example of miR-335* probe is commercialized by Applied Biosystems™ (Ref Cat. # 4427975 hsa-miR-335*).

In some embodiment, said ligand is a primer which specifically hybridizes to a biomarker comprising a nucleic acid selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, a variant, a fragment and a complementary sequence thereof.

The term **"primer"** is meant for short nucleic acid molecules, such as a DNA oligonucleotide, which can be annealed to a complementary target nucleic acid molecule by nucleic acid hybridization to form a hybrid between the primer and the target nucleic acid strand. A primer can be extended along the target nucleic acid molecule by a polymerase enzyme. Therefore, primers can be used to amplify a target nucleic acid molecule. Primer pairs can be used for amplification of a nucleic acid sequence, for example, by PCR, real-time PCR, or other nucleic-acid amplification methods known in the art. Methods for preparing and using primers are described for example, in Sambrook et al. ((1989) Molecular Cloning: A Laboratory Manual, Cold Spring Harbor, New York). Said primer may be a sense primer and/or an antisense primer, said sense primer comprising successive 15 to 40 nucleotides of a nucleotide sequence that is identical to or substantially identical (i.e. at least 75%, 80%, 90%, 95% or 98% identical) to the nucleotide sequence SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14 or SEQ ID NO: 15 and said antisense primer comprising successive 15 to 40 nucleotides of a nucleotide sequence that is complementary to a nucleotide sequence that is identical to or substantially identical (i.e. at least 75%, 80%, 90%, 95% or 98% identical) to the nucleotide sequence SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14 or SEQ ID NO: 15. Preferably, primers may be less than 200, 150, 100, or preferably 50 nucleotide long.

A primer according to the invention may comprise, consist or consist essentially of a nucleotide sequence SEQ ID NO: 16 or SEQ ID NO: 18 or may comprise, consist or consist essentially of a nucleotide sequence within or on the vicinity of sequence SEQ ID NO: 16 or SEQ ID NO: 18.

Primers may also comprise sequence SEQ ID NO: 16 or SEQ ID NO: 18 located at their extreme 3' end or their extreme 5' end, and further comprise unrelated sequences or sequences derived from the mentioned nucleotide sequences, but comprising mismatches.

Examples of MEST primers are commercialized by Applied Biosystems™ (MEST(mouse) Cat. # 4331182 Mm00484993_m1; MEST (human) Cat. # 4331182 Hs00853380_g1). An example of miR-335 primer is commercialized by Applied Biosystems™ (Ref Cat. # 4427975 Mm-miR-335). An example of miR-335* primer is commercialized by Applied Biosystems™ (Ref Cat. # 4427975 hsa-miR-335*).

According to another embodiment, said ligand is an antibody or a binding partner of said biomarker.

A **"binding partner"** refers to a molecule (peptidyl or non-peptidyl) that interact directly with a target molecule such as a nucleic acid of sequence SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14 or SEQ ID NO: 15 or the amino acid sequence of SEQ ID NO: 4, SEQ ID NO: 9, SEQ ID NO: 10 or SEQ ID NO: 20. Preferably said binding partner is a protein partner or a fusion protein partner or its binding domain or an antibody, antibody variants, antibody-derived molecules. Preferably said binding partner is a nucleic acid, RNA aptamers or ribozymes.

The term **"antibody"** ("Ab") or **"immunoglobulin"** (Ig) refers to any form of a peptide, polypeptide derived from, modeled after or encoded by, an immunoglobulin gene, or fragment thereof, that is capable of binding an antigen or epitope. See, e.g., IMMUNOBIOLOGY, Fifth Edition, C. A. Janeway, P. Travers, M., Walport, M. J. Shlomchiked., ed. Garland Publishing (2001). The term "antibody" is used herein in the broadest sense, and encompasses monoclonal, polyclonal or multispecific antibodies, minibodies, heteroconjugates, diabodies, triabodies, chimeric, antibodies, synthetic antibodies, antibody fragments, and binding agents that employ the complementarity determining regions (CDRs) of the parent antibody, or variants thereof that retain antigen binding activity. An immunospecific antibody may be obtained by administering a given polypeptide to an animal followed by recovery of the antibodies produced by said animal by way of extraction from its bodily fluids. A variant of said polypeptide, or host cells expressing said polypeptide may also be administered to the animal.

An antibody according to the invention may specifically recognize a polypeptide having a sequence selected from the group consisting of SEQ ID NO: 4, SEQ ID NO: 9, SEQ ID NO: 10 and SEQ ID NO: 20.

An antibody according to the invention may for example specifically recognize residues 154 to 168 of sequence SEQ ID NO: 4 such as the antibodies commercialized by Acris Antibodies GmbH (ref: AP23691 PU-N) or by LifeSpan Bioscience (ref: LS-C108098) or may specifically recognize residues 40 to 188 of sequence SEQ ID NO: 4 such as a MEST polyclonal antibody commercialized by Abnova under the reference PAB20303.

An antibody according to the invention may specifically recognize a nucleic acid having a sequence selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15.

The invention also concerns a pair of primers suitable for amplifying a nucleic acid comprising a sequence selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, a variant, a fragment and a complementary sequence thereof, for use for diagnosing or prognosing of a mitochondrial disease.

The invention also concerns a kit comprising means for detecting the amount and/or expression of at least one nucleic acid comprising a sequence selected from the group consisting of SEQ ID NO: 5, SEQ ID NO: 6, SEQ ID NO: 8, SEQ ID NO: 13, SEQ ID NO: 14, SEQ ID NO: 15, a variant and a fragment thereof, and/or the ligand according to the invention and/or the pair of primers according to the invention. Preferably, said kit comprises means for detecting the amount and/or the level of expression a nucleic acid comprising SEQ ID NO: 5 and a nucleic acid comprising SEQ ID NO: 6 or a nucleic acid comprising SEQ ID NO: 6 and a nucleic acid comprising SEQ ID NO: 8 or a nucleic acid comprising SEQ ID NO: 5 and a nucleic acid comprising SEQ ID NO: 8, a nucleic acid comprising SEQ ID NO: 13 and a nucleic acid comprising SEQ ID NO: 6 or a nucleic acid comprising SEQ ID NO: 6 and a nucleic acid comprising SEQ ID NO: 14 or a nucleic acid comprising SEQ ID NO: 15 and a nucleic acid comprising SEQ ID NO: 6 or a nucleic acid comprising SEQ ID NO: 5 and a nucleic acid comprising SEQ ID NO: 13 or a nucleic acid comprising SEQ ID NO: 14 and a nucleic acid comprising SEQ ID NO: 13 or a nucleic acid comprising SEQ ID NO: 15 and a nucleic acid comprising SEQ ID NO: 13 or a nucleic acid comprising SEQ ID NO: 13 and a nucleic acid comprising SEQ ID NO: 8 or a nucleic acid comprising SEQ ID NO: 8 and a nucleic acid comprising SEQ ID NO: 14 or a nucleic acid comprising SEQ ID NO: 15 and a nucleic acid comprising SEQ ID NO: 8 or a nucleic acid comprising SEQ ID NO: 5, a nucleic acid comprising SEQ ID NO: 6 and a nucleic acid comprising SEQ ID NO: 8 or a nucleic acid comprising SEQ ID NO: 5, a nucleic acid comprising SEQ ID NO: 6 and a nucleic acid comprising SEQ ID NO: 13 or a nucleic acid comprising SEQ ID NO: 5, a nucleic acid comprising SEQ ID NO: 6 and a nucleic acid comprising SEQ ID NO: 14 or a nucleic acid comprising SEQ ID NO: 5, a nucleic acid comprising SEQ ID NO: 6 and a nucleic acid comprising SEQ ID NO: 15 or a nucleic acid comprising SEQ ID NO: 13, a nucleic acid comprising SEQ ID NO: 6 and a nucleic acid comprising SEQ ID NO: 8 or a nucleic acid comprising SEQ ID NO: 14, a nucleic acid comprising SEQ ID NO: 6 and a nucleic acid comprising SEQ ID NO: 8 or a nucleic acid comprising SEQ ID NO: 15, a nucleic acid comprising SEQ ID NO: 6 and a nucleic acid comprising SEQ ID NO: 8 or a nucleic acid comprising SEQ ID NO: 13, a nucleic acid comprising SEQ ID NO: 14 and a nucleic acid comprising SEQ ID NO: 15 a variant, a fragment thereof.

The kits according to the invention may be used for evaluating the probability of developing a mitochondrial disease, for diagnosis or prognosis of a mitochondrial disease by determining the expression of at least one biomarker of the invention. The kit of the invention may thus comprise a combination of reagents allowing to measure the expression of at least one of the nucleic acid of sequence SEQ ID NO: 1, SEQ ID NO: 2, SEQ ID NO: 3, SEQ ID NO: 5, SEQ ID NO: 6 SEQ ID NO: 7, SEQ ID NO: 8, SEQ ID NO: 11, SEQ ID NO: 12, SEQ ID NO: 13, SEQ ID NO: 14 or SEQ ID NO: 15 or the amino acid sequence of SEQ ID NO: 4, SEQ ID NO: 9, SEQ ID NO: 10 or SEQ ID NO: 20 and optionally the instructions of the manufacturer for use in the diagnosis or prognosis of a mitochondrial disease. Preferably, said kit is designed to measure the level of RNA (notably, mRNA, miRNA) or protein encoded by at least one of the nucleic acid of sequence SEQ ID NO: 1, SEQ ID NO: 2, SEQ ID NO: 3, SEQ ID NO: 5, SEQ ID NO: 6 SEQ ID NO: 7, SEQ ID NO: 8, SEQ ID NO: 11, SEQ ID NO: 12, SEQ ID NO: 13, SEQ ID NO: 14 or SEQ ID NO: 15. Accordingly, it comprises, for each of the biomarkers to be tested, at least one probe or a pair of primers that selectively hybridizes with the transcript of said biomarkers, or with the complement thereof. The kit according to the invention may further comprise at least one control sample.

Although having distinct significances, the terms comprising, **'containing',** and **'consisting of'** were used in an interchangeable way in the description of the invention, and can be replaced one by the other.

The invention will be further described in view of the following examples.

### BRIEF DESCRIPTION OF THE FIGURES

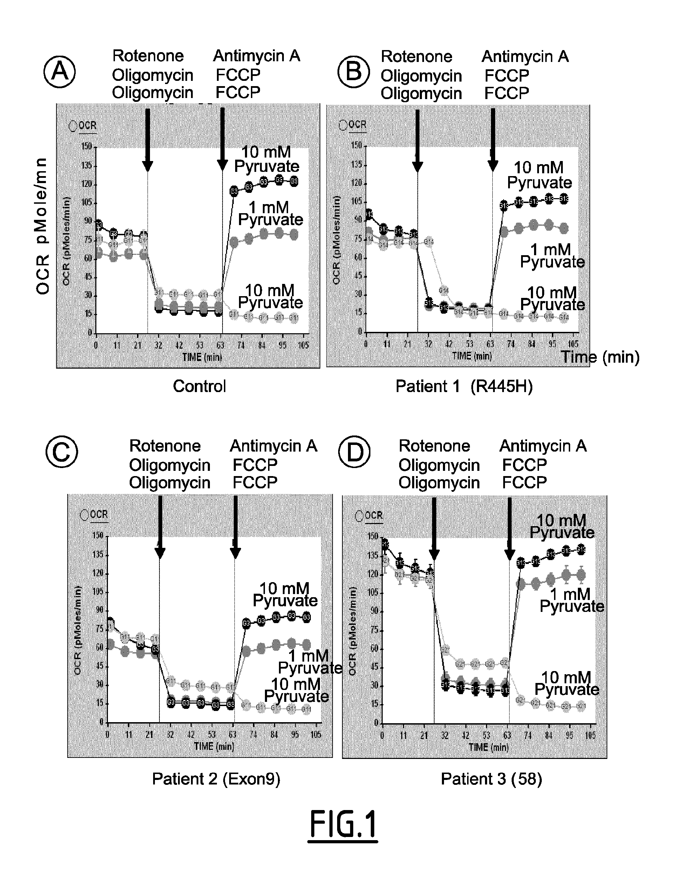

**Figure 1****:** illustrates the characterisation of the biological samples for mitochondrial abnormalities in a sample of (A) a control patient, (B) a Patient 1; (C) a Patient 2; (D) a Patient 3. For example, skin fibroblasts of Dominant Optic Atrophy patients (DOA / mutation in the OPA1 gene) of patients have partial mitochondrial uncoupling comparatively to the control as indicated by the decrease of the RCR (Respiratory Control Ratio) parameter. Control RCR: 13.1; Patient 1 RCR: 7.0; Patient 2 RCR: 8.4 and Patient 3 RCR: 6.9. Decreased of RCR in patient fibroblast is indicative of partial mitochondrial uncoupling. The studies are done by quantifying the oxygen consumption rate using the Sea Horse apparatus.

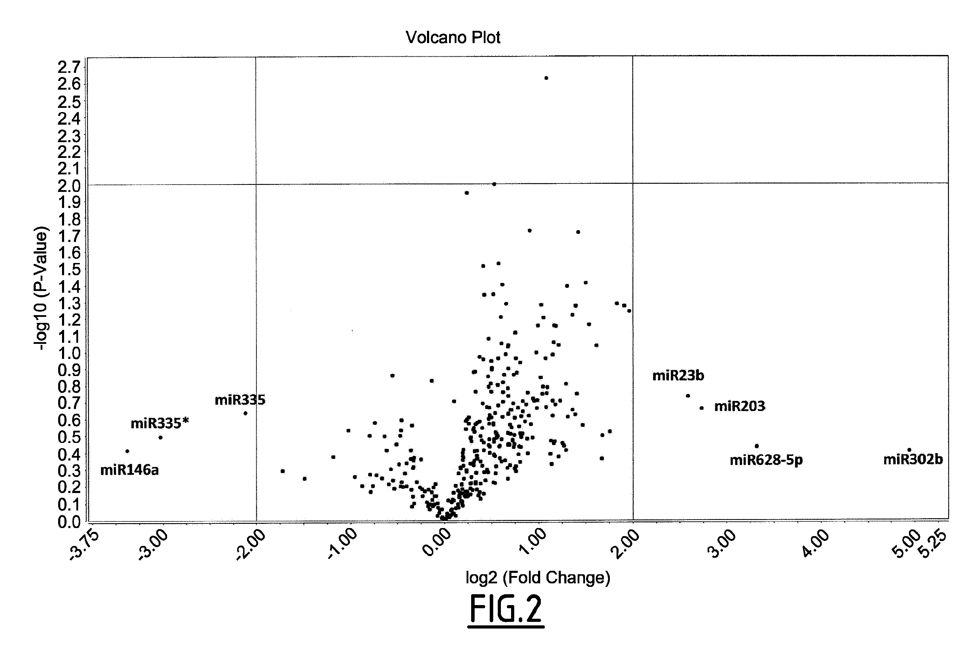

**Figure 2****:** illustrates candidate microRNAs deregulated in DOA patient fibroblasts. The comparative microRNAs profiling are done using 4 DOA patients (OPA1-R445H mutation, OPA1-Exon9 and OPA1-D58 mutant) versus 2 controls by the TLDA technology from Applied Biosystems. The results are shown using the Vulcano Plot.

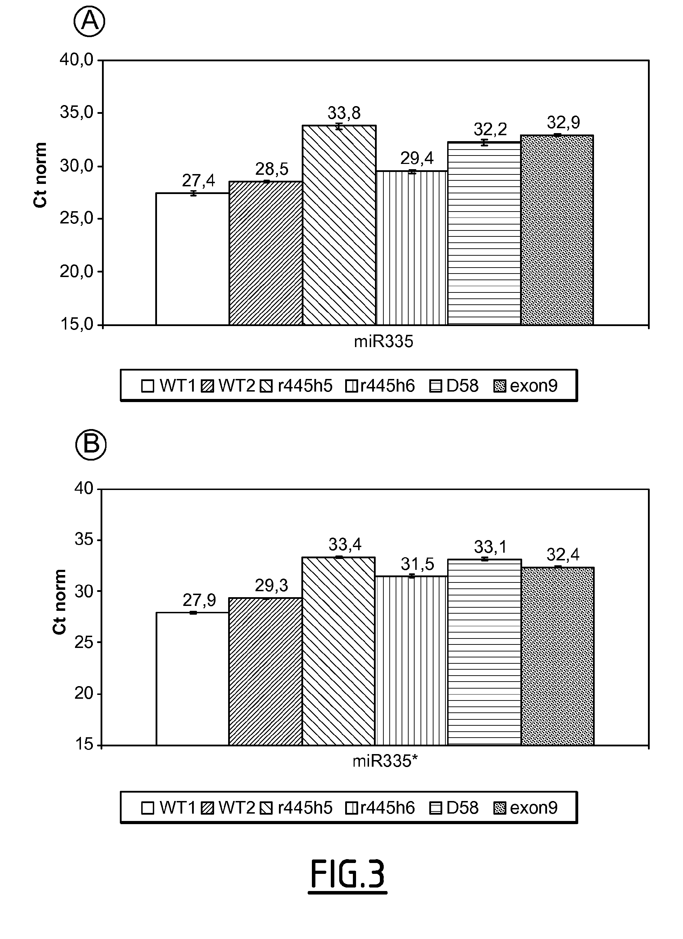

**Figure 3****:** illustrates the validation of the down regulation of the miR-335 and miR-335* in fibroblasts of DOA patients. The analyses are done using Taqman technology from Applied Biosystems. Histogram of the obtained Ct values is presented. The U6 RNA is used for normalization (Ct at 20).

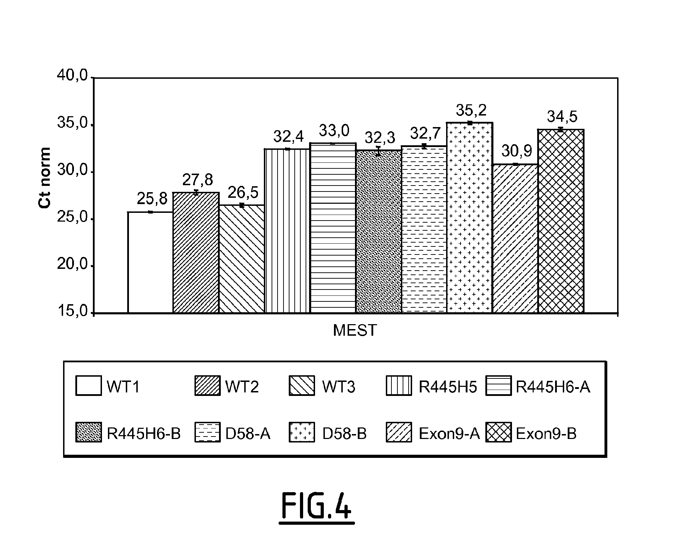

**Figure 4****:** illustrates the validation of the down regulation of the MEST transcript in DOA patient fibroblasts. The analyses are done using Taqman technology from Applied Biosystems. Histogram of the obtained Ct values is presented. The RPLPO mRNA is used for normalization (Ct at 19)

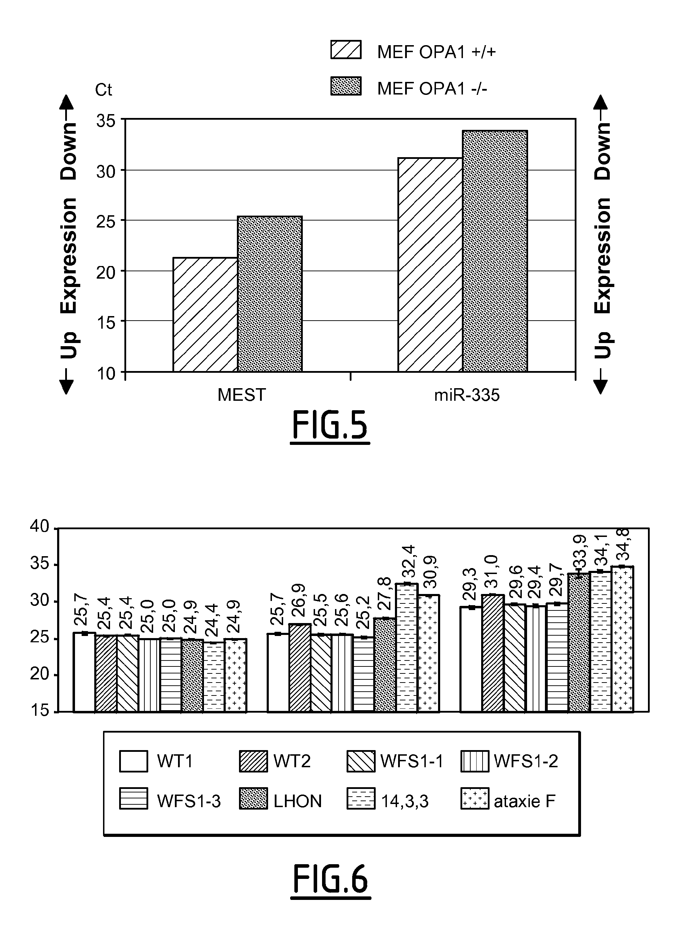

**Figure 5****:** illustrates the down regulation of MEST transcript and miR-335 in OPA1 Knock-Down mouse embryonic fibroblasts (MEF OPA1-/-). The analyses are done using Taqman technology. Histogram of the obtained Ct values is presented. The GAPDH mRNA is used for normalisation.
**Figure 6****:** illustrates the down regulation of MEST transcript and miR-335 expression in skin fibroblasts of patients having other human mitochondrial pathologies: one patient with Leber's Hereditary Optic Neuropathy (LHON, mtDNA mutations), one patient with Friedreich ataxia (FXN mutation), one patient with an 14-3-3eta dominant negative mutation (leading optic atrophy) and patients with Wolfram syndrome (WSF1 mutations / endoplasmic reticulum dysfunction) . The analyses are done using Taqman technology from Applied Biosystems. Histogram of the obtained Ct value is presented. The RPLPO mRNA is used for normalization.

### EXAMPLES

### EXAMPLE 1:

### Materials and Methods:

### Cell cultures

The cells are cultured in DMEM complemented with 10% FBS, Glutamax x1 and sodium pyruvate 1mM, at 37°C and 5% CO2.

DMEM: Dulbecco's Modified Eagle Medium X1with 4.5g/L D-Glucose (GIBCO by life technologies ref: 11960-044)

FBS: Fetal Bovine Serum (GIBCO by life technologies ref: 16000)

Glutamax: X100 (GIBCO by life technologies ref 35050-038)

Sodium pyruvate: 100mM (GIBCO by life technologies ref : 11360)

Fibroblast cells are seeded in XF 96-well microplates (SeaHorse Bioscience) at 30x10³ cells/well in 200 µl of complemented DMEM without antibiotics and incubated for 24h at 37 °C in 5% CO₂ atmosphere.

The next day, cells are washed once with 200µl of XF medium (purchased at SeaHorse Biosciences) complemented with sodium pyruvate and 25mM glucose.

### Oxygen Consumption Rate quantification

Assays are initiated by addition of 180 µL of the complemented XF medium prewarmed at 37°C and cells are incubated at 37°C for 30 mn. The plate is analysed for Oxygen consumption rate (OCR) by the SeaHorse XF96 Extracellular Flux Analyser (SeaHorse Bioscience, Billerica, MA, USA)

The SeaHorse apparatus use cartridge which contains fluorescent probes allowing the measurement of mitochondrial respiration and glycolysis in cells, and two pores A and B for compounds injections 10x in 20 µl).The fluorescent probes must be rehydrated 24h before use, by incubating at 37°C in 200 µL of XF calibrant solution.

After an initial step of calibration, each OCR quantification (Oxygen Consumption Rate) is obtained by 1 mn mixing and 3 mn measuring. The OCR is measured, first, before injection of compound(s) leading the basal respiration, in second after compound injection through the pore A and in third after compound injection through the pore B.

Experiments are done by sequential injection of 2.5 µ/ml Oligomycin through pore A and of 3 µM FCCP (uncoupler carbonyl cyanide p-(trifluoromethoxy)phenylhydrazone) through pore B. Oligomycin (inhibitor of complex V of mitochondrial Electron Transfer Chain) allows us measuring OCR of the nonphosphorylated /coupled respiration. FCCP (uncoupling compound of Electron Transfer Chain) allows us measuring OCR of the maximal uncoupled respiration.

Others experiments are done by sequential injection of 1 µM Rotenone and 10 µM Antimycin A. Injection of Rotenone (inhibitor of complex I) and Antimycin A (inhibitor of complex III), inhibiting totally the Electron Transfer Chain, allows us measuring the non-mitochondrial OCR.

All chemical compounds were purchased at Sigma-Aldrich.

Standardization of the experiment was obtained after quantification of proteins content using BCA Protein Assay kit (Pierce Biotechnology): At the end of the respiration experiment, cells were washed once with 200 µl of cold PBS and lysed with 1% SDS (Sodium dodecyl sulphate, Sigma-Aldrich). An aliquot of sample was assayed and protein content defined by using a BSA (Bovine serum albumin) standard curve.

The respiratory control ratio (RCR) is calculated as the ratio of the FCCP uncoupled to the oligomycin insensitive respiration.

### Results:

**Table 1:**

| *Crude OCR measure* | | | | |
|---|---|---|---|---|
| OCR pmoles/mn | Basal | Olygomycin | FCCP 10 mM Pyruvate | AntimycinA |
| Control | 78 | 21 | 124 | 12.5 |
| Patient 1 | 79 | 19.5 | 108 | 12 |
| Patient 2 | 59 | 16.5 | 86 | 10.5 |
| Patient 3 | 121.5 | 31 | 140.5 | 13 |

**Table 2:**

| OCR pmoles/mn | Olygomycin insensitive respiration (Olygomycin - AntimycinA) | FCCP Uncoupled respiration (FCCP- AntimycinA) |
|---|---|---|
| Control | 8.5 | 111.5 |
| Patient 1 | 7.5 | 96 |
| Patient 2 | 6 | 75.5 |
| Patient 3 | 18 | 122 |

**Table 3:**

| *Respiratory parameters normalized to mg protein:* OCR is expressed in nmoles O₂/mn/mg protein | | |
|---|---|---|
| OCR | Olygomycin insensitive respiration | FCCP Uncoupled respiration |
| Control | 1.06 | 13.95 |
| Patient 1 | 1.5 | 10.55 |
| Patient 2 | 0.9 | 7.55 |
| Patient 3 | 2 | 13.4 |

**Table 4**

| | RCR |
|---|---|
| Control | 13.1 |
| Patient 1 | 7.0 |
| Patient 2 | 8.4 |
| Patient 3 | 6.9 |

Decrease of RCR in patient's fibroblast is indicative of a partial mitochondrial uncoupling

### EXAMPLE 2:

### Materials and Methods:

### A. Samples

The analysis is made from skin fibroblast isolated from four patients: Two patients having a mutation in the OPA1 gene leading to a haplo-insufficiency situation with reduction of 50% in the amount of OPA1 protein ((i) a mutation (c.2708delTTAG) leading deletion of the last 58 residues of OPA1 protein (Δ58) corresponding to sample 6 and (ii) a mutation in intronic region (c.984G>A) leading deletion of the Exon9 corresponding to sample 5) and two patients having a dominant negative mutation (R445H) in the OPA1 gene leading product that acts antagonistically to the wild-type allele (samples 3 and 4). Two skin fibroblasts isolated from healthy donors are used as controls (samples 1 and 2).

### B: Cell Cultures

The cells are cultured in DMEM complemented with 10% FBS, Glutamax x1, sodium pyruvate 1mM, 100 IU/ml penicillin and 100 µg/ml streptomycin at 37°C and 5% CO2.

DMEM: Dulbecco's Modified Eagle Medium X1with 4.5g/L D-Glucose (GIBCO by life technologies ref: 11960-044).

FBS: Fetal Bovine Serum (GIBCO by life technologies ref: 16000).

Glutamax:X100 (GIBCO by life technologies ref 35050-038).

Sodium pyruvate: 100mM (GIBCO by life technologies ref : 11360).

Antibiotics: penicillin- streptomycine (500x) (ROCHE diagnostic GmbH ref: 11074440001).

At 50 to 80% cell confluence, the medium is aspirated and the cell washed with PBS. Trypsin in PBS is added (0.10-0.25%) to cell detachment. Medium, containing serum to inactivate the trypsin is added and cells transferred in tube to pellet cells by centrifugation (300 x g for 5 min). Supernatant is completely aspirated and the pellet is washed using PBS x1. After a second centrifugation, the supernatant is aspirate and the cell pellet is frozen (-80 °C).

### C: RNA preparations

*1- Total RNA* is extracted from cell pellets (from 1-5 10⁶ cells) using the Norgen total RNA Purification kit (Norgen Biotek, Thorold, ON,Canada Cat# 17200) according to the manufacturer's instructions (PI17200-28, 2009).
*2: Genomic DNA* was removed by DNAse I treatment using Turbo DNAse-free kit from Ambion (Ref: AM 1907) and pure total RNAs were recovered by using RNA Clean-up kit from Norgen Biotek (Ref:23600) according to the manufacturer's instructions.

The DNAse I treatment applied is a routine DNAse treatment according to the manufacturer's instructions (publication number 1907M revision G, 05 October 2012); a rigorous DNAse treatment can also be applied if necessary.

The RNA Clean-up protocol used is the one for a Non-Phenol /Guanidine-Based RNA isolation Method according to the manufacturer's instructions (PI23600-10 dated 2010).

### C: Quality control of RNA preparations:

Quantitative analysis is achieved by Thermo Scientific NanoDrop™ 1000 Spectrophotometer.

The RNA is analysed using Agilent RNA 6000 Nano Kit (ref 5067-1511) according to the manufacturer's instructions (08/2006, G2938-90034).

Qualitative analysis of RNAs is achieved on nano labchip processed by the 2100 Expert Bioanalyzer (Agilent 2100 Bioanalyzer; Software B.02.07.SI532) according to manufacturer. The labchip consists of a microfluidic device designed for electrophoretic separation of nucleic acids and detection by a fluorescent dye.

The quality and grade of RNAs are expressed by the RNA Integrity Number (RIN) on a quantitative scale of 1 to 10. RNA preparations are qualified when the RIN values are up to 8.

### D. Human microRNA profiling from RNA samples

miRNA profiling is performed using The TaqMan® Custom Array technology using micro fluidic cards from Applied Biosystems. Card allows performing 384 simultaneous real-time PCR reactions. Each card has 8 sample-loading ports, each connected to 48 reaction wells. The protocol involves pipetting the sample (cDNA obtained by Reverse Transcription of RNA sample) into the ports and briefly centrifuging. Each well contains, for specific miRNA detection, a pair of unlabeled PCR primers specific to the target and a probe with a fluorescent dye (FAM) on the 5' end and a non-fluorescent quencher on the 3' end enabling to label PCR products during thermal cycling. The cards are running on an Applied Biosystems ViiA 7™ Real-Time PCR measuring the exponential accumulation of fluorescent signal on each well. Results are expressed in threshold cycles (Ct), whose Ct is the intersection between the amplification curve and a threshold line. The Ct is a relative measure of the concentration of the target.

### MicroRNA Reverse Transcription (RT) to single-stranded cDNAs:

Reverse transcription (RT) is performed according to the manufacturer's instructions using the Megaplex™ RT Human Pool A version v2.1 (ref. Applied 4399966) and Megaplex™ RT Human Pool B V3.0 (ref. Applied 4399968) containing microRNA specific primers RT primers (for 671 unique microRNAs) using the TaqMan® MicroRNA RT Kit (ref. Applied 4366597) containing enzymatic and buffer components.

In a microcentrifuge tube, prepare the RT master mix on ice by scaling the volumes listed in table 5 below to the desired number of RT reactions:

**Table 5:**

| | |
|---|---|
| Megaplex™ RT Primers | 0.8 µl |
| dNTPs (100 mM) | 0.2 µl |
| MultiScribe™ Reverse Transcriptase (50 U/µL) | 1.5 µl |
| RT Buffer | 0.8 µl |
| MgCl2 (25 mM) | 0.9 µl |
| RNase Inhibitor (20 U/µL) | 0.1 µl |
| Nuclease-free water | 0.2 µl |
| Total | 4.5 µl |

For one RT reaction, add 3 µL (350 to 1000 ng) of the RNA sample into a 0.2 ml PCR tube containing 4.5 µl of the RT master mix. The Reverse transcription (Megaplex™ RT product) is performed using the thermocycler MJ research PTC-200 DNA engine (Bio-Rad Laboratories Hercules CA USA) The run method consists in 40 thermal cycles (2 min at 16°C, 1 min at 42°C, 1 sec at 50°C) followed by 5 min at 85°C and held at 4°C.

*miRNA profiling by Real-time PCR reactions using MicroRNA Array cards.*

miRNA profiling is performed according to manufacturer using the TaqMan® Array Human MicroRNA A+B Cards Set v3.0 (Ref. Applied 4444913) containing specific PCR primers and fluorescent probes and the TaqMan® Universal PCR Master Mix, No AmpErase@ UNG 2X (Ref. Applied 4324018) containing enzymatic and buffer components.

The following components are ombineb in a 1.5-ml tube. The volumes for one sample are listed in table 6 below.

**Table 6:**

| | |
|---|---|
| TaqMan® Universal PCR Master Mix, No AmpErase@ UNG 2X | 450 µL |
| Megaplex™ RT product | 6 µL |
| Nuclease-free water | 444 µL |
| Total | 900 µL |

Dispense 100 µL of the PCR reaction mix into each port of the TaqMan MicroRNA Arrays.

Centrifuge at 260 x g for 1 min and seal the array using a TaqMan® Array Micro Fluidic Card Sealer.

Run on an Applied Biosystems ViiA 7™ Real-Time PCR. The run method consists of previous denaturing stages at 50°C for 2 min and 95°C for 10mn followed by 40 thermal repeated cycles (15 seconds at 95°C, 1 min at 60°C).

The resulted Ct values are normalized using the u6 RNA. Analyses are done using the DataAssist™ v3.0 Software from Aplied Biosystems. The Software is a data analysis tool for sample comparison when using the comparative CT (ΔΔCT) method (Livak and Schmittgen, 2008) for calculating relative quantitation of gene expression. Results are expressed using a Vulcano plot showing Log2 Fold Change plotted against p value.

### Results

### Quality control of RNA preparations

For each sample tested, the two ribosomal RNA peaks corresponding to RNA18S and RNA28S are clearly observable in the electropherogram of the ladder confirming a successful sample run.

The RNA concentration, the ribosomal ratio and the RNA Integrity Number (RIN) calculated for each sample are shown in table 7 hereunder. The RNA Integrity Number (RIN) allows the classification of total RNA based on a numbering system from 1 to 10, with 1 being the most degraded and 10 being the most intact. An RNA sample with a RIN value of 8 as for sample 4 is indicative of an acceptable quality RNA. An RNA sample with a RIN value of 10 as for samples 1, 2, 3, 5 and 6 4 is indicative of a highly intact RNA sample.

**Table 7: Quantitative RNA analysis of samples 1 to 6 corresponding to the total RNA sample of Fibroblasts of healthy subjects (Samples 1 and 2), or to the total RNA sample of Fibroblasts of patient having a mitochondrial default caused by a mutation in R445H of OPA1 gene (sample 3 and 4), or in Exon 9 of OPA1 gene (sample 5) or a deletion of the GTPase effector domain of OPA1 protein (mutation Delta58) (sample 6).**

| Samples | 1 | 2 | 3 | 4 | 5 | 6 |
|---|---|---|---|---|---|---|
| RNA concentration (ng/µl) | 350 | 355 | 180 | 688 | 317 | 317 |
| RNA integrity number (RIN) | 9.9 | 9.4 | 9.8 | 8 | 9.9 | 9.9 |

### Human microRNA profiling from RNA samples

In order to identify the variations in the miRNA transcriptome induced by a mitochondrial default notably by a mutation of OPA1 by comparative human miRNAs profiling between WT samples versus DOA samples have been done and results analysed by a volcano Plot study (figure 2) between total RNA samples from WT fibroblasts (samples 1 or 2) and total RNA samples from fibroblasts of patient having a mitochondrial default (samples 3 to 6). Values that display largest magnitude fold changes (hence being left- (down-exression) or right- (up-expression) of center) have been identified in the left side miR146a, miR335* and miR335 as miRNAs candidates having a down-expression in DOA samples and in the right left miR23b, miR203, miR628-5p and miR302b as miRNAs candidates having an up-expression. Candidates has been studied by individual transcriptomic analysis for confirmation leading confirmation for the miR335 and miR335* (see example 4) and not for the other candidates.

### EXAMPLE 4: Expression quantification of miR335 and miR335* microRNAs

### Materials and Methods:

### Samples

The analysis is made from skin fibroblast isolated from four patients: Two patients having a mutation in the OPA1 gene leading to a haplo-insufficiency situation with reduction of ∼50% in the amount of OPA1 protein ((i) a mutation (c.2708delTTAG) leading deletion of the last 58 residues of OPA1 protein (D58 sample see figure 3) and (ii) a mutation in intronic region (c.984G>A) leading deletion of the Exon9 (exon9 sample see figure 3) and two patients having a dominant negative mutation (R445H) in the OPA1 gene leading product that acts antagonistically to the wild-type allele (R445H5 and R445H6 samples see figure 3). Two skin fibroblasts samples isolated from two healthy donors are used as control (wt1 and wt2 samples see figure 3).

The Cell Cultures conditions, RNA preparations and Quality control of RNA preparations are performed as described in Materials and Methods example 2

miRNA quantification is performed using The TaqMan® technology from Applied Biosystems. The reaction medium contains a pair of unlabeled PCR primers specific to the target and a probe with a fluorescent dye (FAM) on the 5' end and a non-fluorescent quencher on the 3' end enabling to label PCR products during thermal cycling. The reaction is running on an Applied Biosystems ViiA 7™ Real-Time PCR measuring the exponential accumulation of fluorescent signal. Result is expressed in threshold cycles (Ct), whose Ct is the intersection between the amplification curve and a threshold line. The Ct is a relative measure of the concentration of the target.

### 1: MicroRNA Reverse Transcription (RT) to single-stranded cDNAs:

Reverse transcription (RT) is performed according to manufacturer using the TaqMan® MicroRNA Reverse Transcription Kit (Ref; Applied 4366596) containing enzymatic and buffer components.

In a microcentrifuge tube, prepare the RT master mix on ice by scaling the volumes listed in table 8 below to the desired number of RT reactions:

**Table 8:**

| | |
|---|---|
| 100mM dNTPs | 0.15 µL |
| MultiScribe™ Reverse Transcriptase, 50 U/µL | 1.00 µL |
| 10X Reverse Transcription Buffer | 1.50 µL |
| RNase Inhibitor, 20 U/µL | 0.19 µL |
| Nuclease-free water | 4.16 µL |
| Total volume | 7.00 µL |

For one RT reaction, add 5 µL (200 ng) of the RNA sample and 3 µl of a specific 5X RT primer into a 0.2 ml PCR tube containing 7 µl of the RT master mix. The Reverse transcription (Megaplex™ RT product) is performed using the thermocycler MJ research PTC-200 DNA engine (Bio-Rad Laboratories Hercules CA USA). The run method consists in the successive incubations: 30 min at 16°C, 30 min at 42°C followed by 5 min at 85°C and held at 4 °C.

### 2: miRNA quantification by Real-time PCR (qPCR).

miRNA quantification is performed according to manufacturer using the TaqMan® Universal PCR Master Mix, No AmpErase@ UNG 2X (Ref. Applied 4324018) containing enzymatic and buffer components. The qPCR is performed in triplicate.

In a micro-tube, prepare PCR reaction mix on ice by scaling the volumes listed in table 9 below to the desired number of PCR reactions:

**Table 9:**

| | |
|---|---|
| TaqMan® Universal PCR Master Mix, No AmpErase® UNG 2X | 10 µL |
| RT product | 1.5 µL |
| Taqman probe-primers mix | 1 µl |
| Nuclease-free water | 7.5 µL |
| Total | 20 µL |

Applied Biosystems Taqman probe-primers mix references:
- miR-335: Cat. # 4427975 hsa-miR-335
- miR-335*: Cat. # 4427975 hsa-miR-335*
- U6: Cat. # 4427975 U6snRNA

The 20 µl mix is transferred onto wells of 96-well plate. The plate is then sealed and centrifuged briefly.

The plate is loaded into the instrument and ran on the Applied Biosystems ViiA 7™ Real-Time PCR. The run method consists of previous denaturing stages at 50°C for 2 min and 95°C for 10mn followed by 40 thermal repeated cycles (15 seconds at 95°C, 1 min at 60 °C).

The detection and quantification of RNA transcripts is based on the fluorescence emitted from the molecule probe at real time. The detection occurs during the accumulation of the PCR product with each cycle of amplification through an exponential curve. Ct (Threshold cycle) values are assigned to each sample. The Ct is the exact cycle that crosses the threshold (The threshold is a horizontal line drawn through the amplification curve at which significant and specific amplification occurs in early exponential curve). The Ct values are normalized using U6 RNA quantification.

The results are presented through histograms of the obtained normalized Ct for each sample.

### Results

Figure 3 shows differential expression of miR-335 and miR-335* between DOA patient and control samples.

For miR-335, the Ct values of DOA patient samples varies from 29.4 (R445H6 sample) to 33.8 (R445H5 sample). The Ct values of healthy donors are 27.4 and 28.5. The results indicates that differential Ct values between DOA and control samples varies from 0.9 (29.4 - 28.5) and 6.4 (33.8 - 27.4). A differential Ct of 1 corresponding to a doubling of transcript RNA (log2 scale), the fold change between DOA and control samples varies from about 1.9 (2^{0.9}) to 84 (2^{6.4}).

For miR-335*, the Ct values of DOA patient samples varies from 31.5 (R445H6 sample) to 33.4 (R445H5 sample). The Ct values of healthy donors are 27.9 and 29.3. The results indicates that differential Ct values between DOA and control samples varies from 2.2 (31.5 - 29.3) and 5.5 (33.4 - 27.9). A differential Ct of 1 corresponding to a doubling of transcript RNA (log2 scale), the fold change between DOA and control samples varies from about 4.6 (2^{2.2}) to 45(2^{5.5}).

The results indicates an under-expression of miR-335 and miR-335* in the DOA samples.

### EXAMPLE 5: Demonstration of a down regulation of the MEST transcript in DOA patient fibroblasts.

The miR335 and miR335* being localized in the intron 1 of the MEST gene, expression analyze of the MEST transcript has been done.

### Materials and Methods:

### Samples

The analysis is made from skin fibroblast isolated from four patients: Two patients having a mutation in the OPA1 gene leading to a haplo-insufficiency situation with reduction of 50% in the amount of OPA1 protein ((i) a mutation (c.2708delTTAG) leading deletion of the last 58 residues of OPA1 protein (Δ58) (D58A and D58B prepared from two independent cell cultures) and (ii) a mutation in intronic region (c.984G>A) leading deletion of the Exon9 (Exon 9-A and Exon 9-B prepared from two independent cell cultures) and two patients having a dominant negative mutation (R445H) in the OPA1 gene leading product that acts antagonistically to the wild-type allele (R445H5 and R445H6; R445H6-A and R445H6-B prepared from two independent cell cultures of the R445H6 patient sample). Three skin fibroblasts samples isolated from healthy donors (WT1, WT2, WT3) are used as control.

The Cell Cultures conditions, RNA preparations and Quality control of RNA preparations are performed as described in Materials and Methods example 2

### Quantification of the MEST transcript

mRNA quantification is performed using The TaqMan® technology from Applied Biosystems

### a: MEST mRNA Reverse Transcription (RT) to single-stranded cDNA:

Reverse transcription (RT) is performed according to manufacturer using the SuperScript® VILO™ cDNA Synthesis Kit (Invitrogen life technologies ref : 11754-050) containing enzymatic, buffer and hexaprimer components.

In a micro tube, prepare the RT master mix on ice by scaling the volumes listed in table 11 below to the desired number of RT reactions:

**Table 11:**

| | |
|---|---|
| 5X VILO™ Reaction Mix | 4 µl |
| 10X SuperScriptR Enzyme Mix | 2 µl |
| RNA (1µg) | X µl |
| DEPC-treated water to final volume | 20 µl |

Using the thermocycler MJ research PTC-200 DNA engine (Bio-Rad Laboratories Hercules CA USA), incubate at 25°C for 10 minutes followed by 60 minutes at 42°C. Terminate the reaction at 85°C at 5 minutes. Dilute the reaction sample 5X. Use cDNA in qPCR or store at -20°C until use.

### b: mRNA MEST quantification by Real-time PCR (qPCR):

mRNA quantification is performed according to manufacturer using the TaqMan® Universal PCR Master Mix, No AmpErase@ UNG 2X (Ref. Applied 4324018) containing enzymatic and buffer components. The qPCR is performed in triplicate.

In a micro-tube, prepare PCR reaction mix the on ice by scaling the volumes listed in table 12 below to the desired number of PCR reactions:

**Table 12:**

| | |
|---|---|
| TaqMan® Universal PCR Master Mix, No AmpErase® UNG 2X | 10 µL |
| RT product | 2 µL |
| Taqman probe | 1 µl |
| Nuclease-free water | 7 µL |
| Total | 20 µL |

Applied Biosystems Taqman probe references:
- MEST: Cat. # 4331182 Hs00853380_g1
- RPLPO : Cat. # 4331182 Hs99999902_m1
MEST human: Hs00853380_g1 Probe
Context sequence: CCCACCCCCAACAGGAATTCTATAG (SEQ ID NO: 16)
RPLPO human: Hs99999902_m1 Probe
Context sequence AAATGTTTCATTGTGGGAGCAGACA (SEQ ID NO: 17)

The 20 µl mix is transferred onto wells of 96-well plate. The plate is then sealed and centrifuged briefly.

The plate is loaded into the instrument and ran on the Applied Biosystems ViiA 7™ Real-Time PCR. The run method consists of previous denaturing stages at 50°C for 2 min and 95°C for 10mn followed by 40 thermal repeated cycles (15 seconds at 95°C, 1 min at 60 °C).

The detection and quantification of RNA transcripts is based on the fluorescence emitted from the molecule probe at real time. The detection occurs during the accumulation of the PCR product with each cycle of amplification through an exponential curve. Ct (Threshold cycle) values are assigned to each sample. The Ct is the exact cycle that crosses the threshold (The threshold is a horizontal line drawn through the amplification curve at which significant and specific amplification occurs in early exponential curve). The Ct values are normalized using RPLPO mRNA quantification.

The results are presented through histograms of the obtained normalized Ct for each sample.

### Results

Figure 4 shows differential expression of MEST transcript between DOA patient versus control samples.

The Ct values of DOA patient samples varies from 30.9 (exon9-A sample) and 35.2 (D58-B sample). The Ct values of healthy donors are 25.8 and 27.8. The results indicates that differential Ct values between DOA and control samples varies from 3.1 (30.9 - 27.8) and 9.4 (35.2 - 25.8). A differential Ct of 1 corresponding to a doubling of transcript RNA (log2 scale), the fold change between DOA and control samples varies from about 8.5 (2^{3.1}) to 675(2^{9.4}).

The results indicate an under-expression of the MEST mRNA in the DOA samples.

### EXAMPLE 6: Down regulation of MEST transcript and miR-335 in OPA1 Knock-Down mouse embryonic fibroblasts (MEF OPA 1-/-).

### Materials and methods:

Mouse embryonic fibroblasts (MEF OPA1-/- and MEF OPA1+/+ control) are cultured as described in example 2. RNA preparations, Quality control of RNA samples and Specific RNA quantification are performed as described in examples 2 and 6.

The miR335 microRNA and the MEST mRNA quantification are performed by using the following references probes:
MEST: Cat. # 4331182 Mm00484993_m1
GAPDH: Cat. # 4331182 Mm99999915_g1
miR-335: Cat. # 4427975 Mm-miR-335
U6: Cat. # 4427975 U6snRNA
MEST mouse: Mm00484993_m1 Probe
Context sequence: TCGCTTGCGCAGGATGAGAGAGTGG (SEQ ID NO: 18)
GAPD mouse: Mm99999915_g1 Probe
Context sequence: GTGAACGGATTTGGCCGTATTGGGC (SEQ ID NO: 19)

### Results:

Figure 5 shows differential expression of MEST transcript and miR335 between MEF OPA1-/- and MEF OPA1+/+ (control).

The Ct values of the MEST transcript are about 21.5 and 25.5 respectively for MEF OPA1-/- and MEF OPA1+/+ indicating a differential Ct values of about 4 between MEF OPA1-/- and control. A differential Ct of 1 corresponding to a doubling of transcript RNA (log2 scale), the fold change between MEF OPA1-/- and control samples is about 16 (2⁴).

The Ct values of miR-335 are about 31 and 34 respectively for MEF OPA1-/- and MEF OPA1+/+ indicating a differential Ct values of about 3 between MEF OPA1-/- and control. A differential Ct of 1 corresponding to a doubling of transcript RNA (log2 scale), the fold change between MEF OPA1-/- and control samples is about 8 (2³).

The results indicate an under-expression of the MEST mRNA and miR-335 in the MEF OPA1-/-

### EXAMPLE 7: Down regulation of MEST transcript and miR-335 expression in skin fibroblasts of patients having Leber's Hereditary Optic Neuropathy (LHON, mtDNA mutations), Friedreich ataxia (FXN mutation) and patient having a 14-3-3eta dominant negative mutation.

### Materials and methods:

### Samples

The analysis is made from skin fibroblast isolated from six patients: One patient having Leber's Hereditary Optic Neuropathy (LHON, mtDNA mutations) (LHON sample). One patient having Friedreich ataxia (Friedreich ataxia sample) and one patient having a 14-3-3eta dominant negative mutation (14,3.3eta). Three patients having Wolfram syndrome (WFS) in which Wolframin protein coded by gene WSF-1 is mutated. Wolframin protein is a transmembrane protein, which is located primarily in the endoplasmic reticulum and ubiquitously expressed with highest levels in brain. Two skin fibroblasts samples isolated from two healthy donors (WT1, WT2) are used as control

The Cell Cultures, the RNA extraction, the Quality control of total RNA samples and the Specific RNA quantification are performed as described in Materials and Methods of examples 2 and 6, respectively for the miR335 microRNA and for MEST mRNA quantification. Normalizations are done with u6 for microRNA quantification and RPLPO for mRNA quantification.

### Results

### Quality control of RNA preparations

For each sample tested, the two ribosomal RNA peaks corresponding to RNA18S and RNA28S are clearly observable in the electropherogram of the ladder confirming a successful sample run.

The RNA concentration, the ribosomal ratio and the RNA Integrity Number (RIN) calculated for each sample are shown in table 13 hereunder. The RNI values of samples 1 to 8 being about 10, indicate their high quality.

**Table 13:**

| Samples | 1 | 2 | 3 | 4 | 5 | 6 | 7 | 8 |
|---|---|---|---|---|---|---|---|---|
| RNA concentration (ng/µl) | 123 | 154 | 140 | 168 | 209 | 698 | 547 | 423 |
| RNA integrity number (RIN) | 10 | 10 | 9.9 | 10 | 10 | 10 | 10 | 9.6 |

### Quantification of miR335 microRNA, MEST mRNA and OPA 1 mRNA.

Figure 6 shows differential expression of the MEST transcripts and miR-335 between the LHON patient sample, the Friedreich ataxia patient sample, sample of patient having a14-3-3eta mutation and control samples.

Concerning the LHON sample and the MEST transcript, a differential of Ct between the patient sample and the controls varies from 0.9 (27.8 - 26-9) and 2.1 (27.8 - 25.7) indicating a fold change between of about 1.9 (2^{0.9}) to about 4.2 (2^{2.1}). For the miR-335, a differential of Ct between the patient sample and the controls varies from 2.9 (33.9 - 31) and 4.6 (33.9 - 29.3) indicating a fold change between of about 7.4 (2^{2.9}) to about 24 (2^{4.6}).

Concerning the Friedreich ataxia sample and the MEST transcript, a differential of Ct between the patient sample and the controls varies from 4 (30.9 - 26-9) and 5.2 (30.9 - 25.7) indicating a fold change between of about 16 (2⁴) to about 36 (2^{5.2}). For the miR-335, a differential of Ct between the patient sample and the controls varies from 3.8 (34.8 - 31) and 5.5 (34.8 - 29.3) indicating a fold change between of about 14 (2^{3.8}) to about 45 (2^{5.5}).

Concerning the sample of patient with the 14-3-3eta mutation and the MEST transcript, a differential of Ct between the patient sample and the controls varies from 5.5 (32.4 - 26-9) and 6.7 (32.4 - 25.7) indicating a fold change between of about 45 (2^{5.5}) to about 100 (2^{6.7}). For the miR-335, a differential of Ct between the patient sample and the controls varies from 3.1 (34.1 - 31) and 4.8 (34.1 - 29.3) indicating a fold change between of about 8 (2^{3.1}) to about 27 (2^{4.8}).

The results indicate, as for DOA samples, a down regulation of MEST and miR-335 for LHON and Friedreich ataxia patients and patient having a14-3-3eta mutation.

Indeed, in figure 6, little or no variation of MEST and miR-335 expression is observed for healthy or Wolfram syndrome (WFS) patients. The Wolfram syndrome is more likely described for having an endoplasmic reticulum deficiency. No variation of OPA1 is observed in all patients.

## Claims

1. An *in vitro* method of diagnosis or prognosis of a mitochondrial disease in an individual, which comprises:
a) determining the level of at least one biomarker selected from the group consisting of MEST, microRNA-335, microRNA-335* and a precursor of said microRNA-335 and microRNA-335* in a biological sample from the individual,
b) comparing the level of said at least one biomarker to a control level, and
c) deducing that the individual has developed or is at risk of developing a mitochondrial disease if the level of said at least one biomarker is lower than the control level, or that the individual has not developed or is not at risk of developing a mitochondrial disease if the level of said at least one biomarker is upper than or equal to the control level.

2. The method according to claim 1 wherein the level of MEST biomarker is determined by measuring the level of expression of MEST protein and/or gene.

3. The method according to claim 1 or 2 wherein the level of expression of MEST protein is determined by detecting a polypeptide comprising SEQ ID NO: 9, SEQ ID NO: 10 and/or SEQ ID NO: 20, a variant or a fragment thereof.

4. The method according to any one of claims 1 to 3 wherein the level of expression of MEST gene is determined by detecting a nucleic acid comprising SEQ ID NO: 8, SEQ ID NO: 13, SEQ ID NO: 14, SEQ ID NO: 15, a variant, a fragment, a complementary sequence or a corresponding RNA sequence thereof.

5. The method according to any one of claims 1 to 4 wherein the level of microRNA-335 biomarker or a precursor thereof is determined by detecting a nucleic acid comprising SEQ ID NO: 5, a variant and a fragment thereof.

6. The method according to any one of claims 1 to 5 wherein the level of microRNA-335* biomarker or a precursor thereof is determined by detecting a nucleic acid comprising SEQ ID NO: 6, a variant or a fragment thereof.

7. The method according to any one of claims 1 to 6 wherein the mitochondrial disease is selected from the group consisting of Dominant Optic Atrophy (DOA), Leber's Hereditary Optic Neuropathy (LHON), Friedreich ataxia and Parkinson Disease.

8. The method according to any one of claims 1 to 7 wherein said at least one biomarker is MEST and said mitochondrial disease is DOA, LHON or Friedreich ataxia.

9. The method according to any one of claims 1 to 7 wherein said at least one biomarker is microRNA-335 and said mitochondrial disease is LHON or Friedreich ataxia.

10. The method according to any one of claims 1 to 7 wherein said method comprises the step of determining the level of expression of at least microRNA-335 and microRNA-335* and said mitochondrial disease is DOA.

11. The method according to any one of claims 1 to 10 wherein the biological sample is a tissue or a biological fluid.

12. Use of at least one of MEST, microRNA-335, microRNA-335*, a precursor of said microRNA-335 and microRNA-335*, a variant or a fragment thereof as a biomarker of a mitochondrial disease.

13. The use of claim 12 wherein said biomarker comprises a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 20, a variant and a fragment thereof.

14. A ligand specifically binding to a biomarker selected from the group consisting of MEST, microRNA-335, microRNA-335* and a precursor of said microRNA-335 and microRNA-335*, for use for diagnosing or prognosing of a mitochondrial disease.

15. The ligand of claim 14 wherein said biomarker comprises a sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 20, a variant and a fragment thereof.

16. The ligand of claim 14 or 15 wherein said ligand specifically hybridizes to a biomarker comprising a nucleic acid selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, a variant, a fragment and a complementary sequence thereof.

17. The ligand of claim 14 wherein said ligand is an antibody or a binding partner of said biomarker.

18. A pair of primers suitable for amplifying a nucleic acid comprising a sequence selected from the group consisting of SEQ ID NO: 1, SEQ ID NO: 2, SEQ ID NO: 3, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, a variant, a fragment and a complementary sequence thereof, for use for diagnosing or prognosing of a mitochondrial disease.

19. A kit comprising means for detecting the amount and/or expression of at least one nucleic acid comprising a sequence selected from the group consisting of SEQ ID NO: 5, SEQ ID NO: 6, SEQ ID NO: 8, SEQ ID NO: 13, SEQ ID NO: 14, SEQ ID NO: 15, a variant and a fragment thereof, and/or the ligand according to any one of claim 14 to 17 and/or the pair of primers according to claim 18.
